# EUROPÄISCHE PATENTSCHRIFT

(11) **EP 1 940 807 B1**
(45) Veröffentlichungstag und Bekanntmachung des Hinweises auf die Patenterteilung: **17.06.2015**
(21) Anmeldenummer: 06806216.5
(22) Anmeldetag: 12.10.2006
(51) Int. Cl.: C07D 249/18, C07D 409/12, C07D 409/14, C07D 405/12, C07D 403/12, A61K 31/4192, A61P 3/00

(54) **CARBAMOYLBENZOTRIAZOL-DERIVATE ALS INHIBITOREN VON LIPASEN UND PHOSPHOLIPASEN**
CARBAMOYLBENZOTRIAZOLE DERIVATIVES AS INHIBITORS OF LIPASES AND PHOSPHOLIPASES
DERIVES DE CARBAMOYLE-BENZOTRIAZOL UTILISES COMME INHIBITEURS DE LIPASES ET DE PHOSPHOLIPASES

(30) Priorität: 19.10.2005 DE 102005049953
(43) Veröffentlichungstag der Anmeldung: 09.07.2008
(73) Patentinhaber: SANOFI, 75008 Paris (FR)
(72) Erfinder: PETRY, Stefan, 65926 Frankfurt am Main (DE); ZOLLER, Gerhard, 65926 Frankfurt am Main (DE); TENNAGELS, Norbert, 65926 Frankfurt am Main (DE); MÜLLER, Günter, 65926 Frankfurt am Main (DE)
(74) Vertreter: Essler, Frank
(86) Internationale Anmeldenummer: PCT/EP2006/009857
(87) Internationale Veröffentlichungsnummer: WO 2007/045393

(56) Entgegenhaltungen:
- EP-A- 1 164 421
- EP-A2- 1 164 419
- WO-A-2004/035550
- GB-A- 1 505 699
- JP-A- 62 039 573
- BUTULA I ET AL: "Reactions with 1-benzotriazolecarboxylic acid. VIII. Synthesis of N-hydroxyisocyanate derivatives" CROATICA CHEMICA ACTA, CROATIAN CHEMICAL SOCIETY, ZAGREB, HR, Bd. 73, Nr. 2, 2000, Seiten 569-574, XP002982484 ISSN: 0011-1643

## Beschreibung

Die vorliegende Erfindung betrifft Benzotriazol-derivate der allgemeinen Formel I, deren pharmazeutisch anwendbare Salze und deren Verwendung als Arzneistoffe.

Benzotriazole sind bereits aus den verschiedensten Gebieten bekannt, wie z.B. der Fotochemie (US 4,255,510, Kodak) oder als Orexin Antagonisten (WO 02/090355, SKB). Ferner ist die Synthese zur Herstellung von Benzotriazolen von Katritzky et al. in J. Org. Chem. 1997, 62, 4155-4158 beschrieben. Bekannt sind ferner Carbamate als Lipase-Inhibitoren wie z.B. Shamkant Patkar et al. in Paul Woolley, Steffen B. Petterson (ed), Lipase (1994) 207-227, WO 03/051842 oder WO 2004/035550. Aufgabe der vorliegenden Erfindung ist die Bereitstellung von Verbindungen, die eine Hemmung der endothelialen Lipase bewirken.

Gegenstand der Erfindung sind Carbamoylbenzotriazol-derivate der allgemeinen Formel I 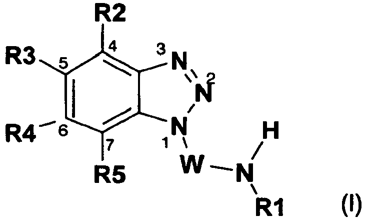 wobei bedeuten:
- W: -(C=O)-, -SO-, -SO₂-;
- R1: X-Aryl, X-Heteroaryl, X-(C₅-C₁₂)-Cycloalkyl oder (C₈-C₁₄)-Bicyclus, wobei Aryl, Heteroaryl, Cycloalkyl oder Bicyclus ein oder mehrfach durch bevorzugt Halogen, (C₁-C₆)-Alkyl, (C₁-C₆)-Alkyloxy, Hydroxy, (C₁-C₆)-Alkylmercapto, Amino, (C₁-C₆)-Alkylamino, di-(C₂-C₁₂)-Alkylamino, mono-(C₁-C₆)-Alkylaminocarbonyl, di-(C₂-C₈)-Alkylaminocarbonyl, (C₁-C₆)-Alkoxycarbonyl, (C₁-C₆)-Alkylcarbonyl, Cyano, Nitro, Trifluormethyl, Trifluormethyloxy, (C₁-C₆)-Alkylsulfonyl, Aminosulfonyl substituiert und durch Y-Aryl, Y-Heteroaryl, Y-(C₃-C₁₂)-Cycloalkyl einfach substituiert sein kann, worin Aryl, Heteroaryl oder Cycloalkyl durch bevorzugt Halogen, (C₁-C₆)-Alkyl, (C₁-C₆)-Alkyloxy, Hydroxy, (C₁-C₆)-Alkylmercapto, Amino, (C₁-C₆)-A)kylamino, di-(C₂-C₁₂)-Alkylamino, mono-(C₁-C₆)-Alkylaminocarbonyl, di-(C₂-C₈)-Alkylaminocarbonyl, (C₁-C₆)-Alkoxycarbonyl, (C₁-C₆)-Alkylcarbonyl, Cyano, Nitro, Trifluormethyl, Trifluormethyloxy, (C₁-C₆)-Alkylsulfonyl, Aminosulfonyl ein bis dreifach substituiert sein kann;
- X: -CH₂-, welches durch Halogen, Methyl oder Hydroxy einfach substituiert sein kann;
- Y: eine Bindung, (C₁-C₃)-Alkylen, -O-, -NH-;
- R2, R3, R4, R5: gleich oder verschieden Wasserstoff, Halogen, (C₁-C₆)-Alkyl, (C₁-C₃)-Haloalkyl, (C₁-C₃)-Alkyloxy-(C₁-C₃)-Alkylen, Hydroxy, Phenoxy, NR6R7, Cyano, Nitro, COOR6, CO-NR6R7, -S-R6, -SO-R6, -SO₂-R6, Aminosulfonyl, Pentafluorsulfanyl, Aryl, Heteroaryl, O-Heteroaryl, (C₃-C₁₂)-Cycloalkyl, CO-R6, CO-NR6R7, O-CO-NR6R7, O-CO-(C₁-C₆)-Alkylen-CO-O-(C₁-C₆)-Alkyl, O-CO-(C₁-C₆)-Alkylen-CO-OH, O-CO-(C₁-C₆)-Alkylen-CO-NR6R7 oder unsubstituiertes oder ein oder mehrfach durch F substituiertes (C₁-C₆)-Alkyloxy;
- R6, R7: gleich oder verschieden Wasserstoff, (C₁-C₆)-Alkyl, Benzyl;
die tautomeren Formen der Verbindungen sowie deren physiologisch verträgliche Salze.

Bevorzugt sind Verbindungen der Formel I, worin
- R2, R3, R4, R5: gleich oder verschieden Wasserstoff, Halogen, (C₁-C₆)-Alkyl, (C₁-C₃)-Haloalkyl, (C₁-C₃)-Alkyloxy-(C₁-C₃)-Alkylen, Hydroxy, Phenoxy, NR6R7, Cyano, Nitro, COOR6, CO-NR6R7, -S-R6, -SO-R6, -SO₂-R6, Aminosulfonyl, Pentafluorsulfanyl, CO-R6, CO-NR6R7, O-CO-NR6R7, O-CO-(C₁-C₆)-Alkylen-CO-O-(C₁-C₆)-Alkyl, O-CO-(C₁-C₆)-Alkylen-CO-OH, O-CO-(C₁-C₆)-Alkylen-CO-NR6R7 oder unsubstituiertes oder ein oder mehrfach durch F substituiertes (C₁-C₆)-Alkyloxy.

Bevorzugt sind Verbindungen der Formel I, worin
- W: -(C=O)- bedeutet.

Weiterhin bevorzugt sind Verbindungen der Formel I, worin
- W: -(C=O)-;
- R1: X-Heteroaryl oder (C₈-C₁₄)-Bicyclus, wobei Aryl, Heteroaryl, Cycloalkyl oder Bicyclus ein oder mehrfach durch bevorzugt Halogen, (C₁-C₆)-Alkyl, (C₁-C₆)-Alkyloxy, Hydroxy, (C₁-C₆)-Alkylmercapto, Amino, (C₁-C₆)-Alkylamino, di-(C₂-C₁₂)-Alkylamino, mono-(C₁-C₆)-Alkylaminocarbonyl, di-(C₂-C₈)-Alkylaminocarbonyl, (C₁-C₆)-Alkoxycarbonyl, (C₁-C₆)-Alkylcarbonyl, Cyano, Nitro, Trifluormethyl, Trifluormethyloxy, (C₁-C₆)-Alkylsulfonyl, Aminosulfonyl substituiert und durch Y-Aryl, Y-Heteroaryl, Y-(C₃-C₁₂)-Cycloalkyl einfach substituiert sein kann, worin Aryl, Heteroaryl oder Cycloalkyl durch bevorzugt Halogen, (C₁-C₆)-Alkyl, (C₁-C₆)-Alkyloxy, Hydroxy, Amino, (C₁-C₆)-Alkylamino, di-(C₂-C₁₂)-Alkylamino, (C₁-C₆)-Alkoxycarbonyl, (C₁-C₆)-Alkylcarbonyl, Cyano, Nitro, Trifluormethyl, Trifluormethyloxy, ein bis zweifach substituiert sein kann;
- X: -CH₂-, welches durch Halogen, Methyl oder Hydroxy einfach substituiert sein kann;
- Y: eine Bindung, -O-, -NH-;
- R2, R3, R4, R5: gleich oder verschieden Wasserstoff, Halogen, (C₁-C₆)-Alkyl, Trifluormethyl, Hydroxy, Amino, Cyano, Phenoxy, (C₁-C₆)-Alkylcarbonyl, (C₁-C₆)-Alkylsulfonyl, Pentafluorsulfanyl, oder unsubstituiertes oder ein oder mehrfach durch F substituiertes (C₁-C₃)-Alkyloxy;
bedeuten,
die tautomeren Formen der Verbindungen sowie deren physiologisch verträgliche Salze.

Besonders bevorzugte Verbindungen der Formel I sind die, in denen
- W: -(C=O)-;
- R1: X-Phenyl, X-Heteroaryl, oder Bicyclus, wobei Aryl, Heteroaryl oder Bicyclus ein oder mehrfach durch bevorzugt Halogen, (C₁-C₆)-Alkyl, (C₁-C₆)-Alkyloxy, Hydroxy, (C₁-C₆)-Alkoxycarbonyl, (C₁-C₆)-Alkylcarbonyl, Cyano, Nitro, Trifluormethyl, Trifluormethyloxy, (C₁-C₆)-Alkylsulfonyl, Aminosulfonyl substituiert und durch Y-Phenyl, Y-Heteroaryl einfach substituiert sein kann, worin Phenyl oder Heteroaryl durch bevorzugt Halogen, (C₁-C₆)-Alkyl, (C₁-C₆)-Alkyloxy, Hydroxy, Amino, (C₁-C₆)-Alkoxycarbonyl, (C₁-C₆)-Alkylcarbonyl, Cyano, Trifluormethyl, Trifluormethyloxy, ein bis zweifach substituiert sein kann;
- X: -CH₂-, welches durch Fluor, Methyl oder Hydroxy substituiert sein kann;
- Y: eine Bindung;
- R2, R3, R4, R5: gleich oder verschieden Wasserstoff, Halogen, (C₁-C₆)-Alkyl, Trifluormethyl, Hydroxy, Amino, Cyano, Phenoxy, (C₁-C₆)-Alkylcarbonyl oder unsubstituiertes oder ein oder mehrfach durch F substituiertes (C₁-C₃)-Alkyloxy;
bedeuten,
die tautomeren Formen der Verbindungen sowie deren physiologisch verträgliche Salze.

Ganz besonders bevorzugte Verbindungen der Formel I sind die, in denen
- W: -(C=O)-;
- R1: X-Phenyl, X-Heteroaryl, oder Bicyclus der Formel Ia 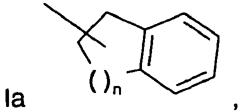 mit n =1 oder 2, wobei Phenyl, Heteroaryl oder Bicyclus der Formel Ia ein oder mehrfach durch bevorzugt Halogen, (C₁-C₆)-Alkyl, (C₁-C₆)-Alkyloxy, Hydroxy, (C₁-C₆)-Alkoxycarbonyl, (C₁-C₆)-Alkylcarbonyl, Cyano, Nitro, Trifluormethyl, Trifluormethyloxy, (C₁-C₆)-Alkylsulfonyl, Aminosulfonyl substituiert und durch Y-Phenyl, Y-Heteroaryl einfach substituiert sein kann, worin Phenyl oder Heteroaryl durch bevorzugt Halogen, (C₁-C₆)-Alkyl, (C₁-C₆)-Alkyloxy, Hydroxy, Amino, (C₁-C₆)-Alkoxycarbonyl, (C₁-C₆)-Alkylcarbonyl, Cyano, Trifluormethyl, Trifluormethyloxy ein bis zweifach substituiert sein kann;
- X: -CH₂-, welches durch Fluor, Methyl oder Hydroxy substituiert sein kann;
- Y: eine Bindung;
- R2, R3, R4, R5: gleich oder verschieden Wasserstoff, Halogen, (C₁-C₆)-Alkyl, Trifluormethyl, Hydroxy, Amino, Cyano, Phenoxy, (C₁-C₆)-Alkylcarbonyl oder unsubstituiertes oder ein oder mehrfach durch F substituiertes (C₁-C₃)-Alkyloxy;
bedeuten,
die tautomeren Formen der Verbindungen sowie deren physiologisch verträgliche Salze.

Weiterhin ganz besonders bevorzugte Verbindungen der Formel I sind die, in denen
- W: -(C=O)-;
- R1: X-Phenyl, X-Heteroaryl oder Bicyclus der Formel Ia 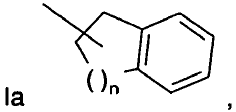 mit n =1 oder 2, wobei Phenyl, Heteroaryl oder Bicyclus der Formel Ia ein oder mehrfach durch bevorzugt Halogen, (C₁-C₆)-Alkyl, (C₁-C₆)-Alkyloxy, Hydroxy, (C₁-C₆)-Alkoxycarbonyl, (C₁-C₆)-Alkylcarbonyl, Cyano, Nitro, Trifluormethyl, Trifluormethyloxy, (C₁-C₆)-Alkylsulfonyl, Aminosulfonyl substituiert und durch Y-Phenyl, Y-Heteroaryl einfach substituiert sein kann, worin Phenyl oder Heteroaryl durch bevorzugt Halogen, (C₁-C₆)-Alkyl, (C₁-C₆)-Alkyloxy, Hydroxy, Amino, (C₁-C₆)-Alkoxycarbonyl, (C₁-C₆)-Alkylcarbonyl, Cyano, Trifluormethyl, Trifluormethyloxy ein bis zweifach substituiert sein kann;
- X: -CH₂-, welches durch Methyl substituiert sein kann;
- Y: eine Bindung;
- R2, R3, R4, R5: gleich oder verschieden Wasserstoff, Halogen, (C₁-C₆)-Alkyl, Trifluormethyl, Hydroxy, Amino, Cyano, Phenoxy, (C₁-C₆)-Alkylcarbonyl oder unsubstituiertes oder ein oder mehrfach durch F substituiertes (C₁-C₃)-Alkyloxy;
bedeuten.

Insbesondere besonders bevorzugte Verbindungen der Formel I sind die, in denen
- W: -(C=O)-;
- R1: X-Phenyl, X-Thienyl, X-Furan, X-Benzothienyl, Indanyl oder Tetrahydronaphtyl, wobei Phenyl, Thienyl, Furan, Benzothienyl, Indanyl oder Tetrahydronaphtyl ein-, zwei- oder dreifach durch F, Cl, Br, Methyl, Ethyl, Isopropyl, Methoxy, Ethoxy, Hydroxy, CO-OCH₃, CO-CH₃, Cyano, Nitro, Trifluormethyl, Trifluormethyloxy substituiert und durch Y-Phenyl, Y-Thienyl, Y-Pyridyl, Y-Pyrazolyl einfach substituiert sein kann, worin Phenyl, Thienyl, Pyridyl oder Pyrazolyl durch bevorzugt F, Cl, Br, Methyl, Methoxy, Hydroxy, Amino, CO-OCH₃, CO-CH₃, Cyano, Trifluormethyl, Trifluormethyloxy ein bis zweifach substituiert sein kann;
- X: -CH₂-, welches durch Methyl substituiert sein kann;
- Y: eine Bindung;
- R2, R3, R4, R5: gleich oder verschieden Wasserstoff, F, Cl, Methoxy, Trifluormethyl, Cyano, Phenoxy;
bedeuten.

Insbesondere ganz besonders bevorzugte Verbindungen der Formel I sind die, worin
- W: -(C=O)-;
- R1: X-Phenyl, X-Thienyl, X-Furan, X-Benzothienyl oder Indanyl, wobei Phenyl, Thienyl, X-Furan, X-Benzothienyl ein oder zweifach durch F, Cl, Br, Methyl, Isopropyl, Methoxy, Cyano, Trifluormethyl substituiert und
durch Y-Phenyl, Y-Thienyl, Y-Pyridyl, Y-Pyrazolyl einfach substituiert sein kann, worin Phenyl durch Cl substituiert sein kann;
- X: -CH₂-, welches durch Methyl substituiert sein kann;
- Y: eine Bindung;
- R2: Wasserstoff, F, Cl;
- R3: Wasserstoff, F, Cl, Methoxy, Trifluormethyl, Cyano, Phenoxy;
- R4: Wasserstoff, F, Cl, Methoxy, Trifluormethyl, Cyano, Phenoxy;
- R5: Wasserstoff, F, Cl;
bedeuten.

Besonders bevorzugt sind auch die Verbindungen der Formel I, worin

Besonders bevorzugt sind auch die Verbindungen der Formel I, worin
- R2, R3, R5: Wasserstoff;
und
- R4: nicht Wasserstoff; oder

- R2, R4, R5: Wasserstoff;
und
- R3: nicht Wasserstoff;
bedeuten.

Besonders bevorzugt sind auch die Verbindungen der Formel I, worin
- R2, R3, R4, R5: Wasserstoff;
bedeuten.

Die Erfindung bezieht sich auf Verbindungen der Formel I, in Form ihrer Salze, Racemate, racemischen Mischungen und reinen Enantiomere sowie auf ihre Diastereomere und Mischungen davon.

Die Alkylreste in den Substituenten R1, R2, R3, R4, R5, R6, R7 können sowohl geradkettig wie verzweigt sein. Beispielhaft seien genannt: Methyl, Ethyl, n-Propyl, i-Propyl, n-Butyl, i-Butyl, t-Butyl, Pentyl, Hexyl, Octyl, Dodecyl. Halogen steht für Fluor, Chlor, Brom oder Iod, besonders für Fluor oder Chlor.

Unter Haloalkyl wird ein durch Halogen ein oder mehrfach substituierter Alkylrest verstanden.

Unter einem Arylrest wird ein Phenyl oder ein Naphthylrest verstanden.

Die Arylreste können ein oder mehrfach mit geeigneten Gruppen substituiert sein, wie z.B.: F, Cl, Br, I, CF₃, OH, OCF₃, NO₂, CN, COOH, COO(C₁-C₆)Alkyl, CONH₂, CONH(C₁-C₆)Alkyl, CON[(C₁-C₆)Alkyl]₂, (C₃-C₁₀)-Cycloalkyl, (C₁-C₁₀)-Alkyl, (C₂-C₆)-Alkenyl, (C₂-C₆)-Alkinyl, O-(C₁-C₆)-Alkyl, CO-(C₁-C₆)-Alkyl, O-CO-(C₁-C₆)-Alkyl, O-CO-(C₁-C₆)-Aryl,
PO₃H₂, SO₃H, SO₂-NH₂, SO₂NH(C₁-C₆)-Alkyl, SO₂N[(C₁-C₆)-Alkyl]₂, S-(C₁-C₆)-Alkyl, S-(CH₂)ₙ-Aryl, S-(CH₂)ₙ-Heterocyclus, SO-(C₁-C₆)-Alkyl, SO-(CH₂)ₙ-Aryl, SO-(CH₂)ₙ-Heterocyclus, SO₂-(C₁-C₆)-Alkyl, SO₂-(CH₂)ₙ-Aryl, SO₂-(CH₂)ₙ-Heterocyclus, SO₂-NH(CH₂)ₙ-Aryl, SO₂-NH(CH₂)ₙ-Heterocyclus, SO₂-N(C₁-C₆)-Alkyl)(CH₂)ₙ-Aryl, SO₂-N(C₁-C₆)-Alkyl)(CH₂)ₙ-Heterocyclus, SO₂-N((CH₂)ₙ-Aryl)₂, SO₂-N((CH₂)ₙ-(Heterocyclus)₂, NH-(CH₂)ₙ-Aryl, NH-(CH₂)ₙ-Heterocyclus, N((C₁-C₆)-Alkyl)(CH₂)ₙ-Aryl, N((C₁-C₆)-Alkyl)(CH₂)ₙ-Heterocyclus,
C(NH)(NH₂), NH₂, NH-(C₁-C₆)-Alkyl, N((C₁-C₆)-Alkyl)₂, NH-CO-(C₁-C₆)-Alkyl, NH-COO-(C₁-C₆)-Alkyl, NH-CO-Aryl, NH-CO-Heterocyclus, NH-COO-Aryl, NH-COO-Heterocyclus, NH-CO-NH-(C₁-C₆)-Alkyl, NH-CO-NH-Aryl, NH-CO-NH-Heterocyclus, Aryl, O-(CH₂)ₙ-Aryl, O-(CH₂)ₙ-Heterocyclus, wobei n = 0 - 6 sein kann, wobei der Arylrest oder Heterocyclische Rest ein bis 3-fach substituiert sein kann mit F, Cl, Br, I, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SO₂-CH₃, COOH, COO-(C₁-C₆)-Alkyl, CONH₂.

Heterocyclus ist ein mono- oder bicyclisches Ringsystem mit 5 bis 12 Ringgliedern, worin mindestens ein Atom im Ringsystem ein Heteroatom aus der Reihe N, O und S ist. Ferner gehören auch Ringsysteme zu dieser Definition, worin der Heterocyclus mit einem Benzolkern kondensiert ist.

Heteroaryl ist ein mono- oder bicyclisches, aromatisches Ringsystem mit 5 bis 12 Ringgliedern, worin mindestens ein Atom im Ringsystem ein Heteroatom aus der Reihe N, O und S ist. Ferner gehören auch Ringsysteme zu dieser Definition, die einen kondensierten Benzolkern enthalten.

Geeignete "Heteroaryl-Ringe" bzw. "Heteroaryl-Reste" sind beispielsweise Benzimidazolyl, Benzofuranyl, Benzothienyl, Berizoxazolyl, Benzthiazolyl, Benztriazolyl, Benztetrazolyl, Benzisoxazolyl, Benzisothiazolyl, Chinolinyl, Furyl, Furazanyl, Imidazolyl, 1H-Indazolyl, Indolyl, 1,2,3-Oxadiazolyl, 1,2,4-Oxadiazolyl, 1,2,5-Oxadiazolyl, 1,3,4-Oxadiazolyl, Pyrimidinyl, Pyrazinyl, Pyrazolyl, Pyridyl, Pyrrolyl, Thiazolyl, 1,2,3-Thiadiazolyl, 1,2,4-Thiadiazolyl, 1,2,5-Thiadiazolyl, 1,3,4-Thiadiazolyl, Thienyl.

Bevorzugte Heteroarylreste sind Thienyl, Furanyl, Benzothienyl, Pyridyl, Pyrazolyl, besonders bevorzugte Thienyl, Furanyl und Benzothienyl, insbesondere bevorzugt Thienyl.

Die Heteroarylreste können ein oder mehrfach mit geeigneten Gruppen substituiert sein, wie z.B.: F, Cl, Br, I, CF₃, OH, OCF₃, NO₂, CN, COOH, COO(C₁-C₆)Alkyl, CONH₂, CONH(C₁-C₆)Alkyl, CON[(C₁-C₆)Alkyl]₂, (C₃-C₁₀)-Cycloalkyl, (C₁-C₁₀)-Alkyl, (C₂-C₆)-Alkenyl, (C₂-C₆)-Alkinyl, O-(C₁-C₆)-Alkyl, CO-(C₁-C₆)-Alkyl, O-CO-(C₁-C₆)-Alkyl, O-CO-(C₁-C₆)-Aryl,
PO₃H₂, SO₃H, SO₂-NH₂, SO₂NH(C₁-C₆)-Alkyl, SO₂N[(C₁-C₆)-Alkyl]₂, S-(C₁-C₆)-Alkyl, S-(CH₂)ₙ-Aryl, S-(CH₂)ₙ-Heterocyclus, SO-(C₁-C₆)-Alkyl, SO-(CH₂)ₙ-Aryl, SO-(CH₂)ₙ-Heterocyclus, SO₂-(C₁-C₆)-Alkyl, SO₂-(CH₂)ₙ-Aryl, SO₂-(CH₂)ₙ-Heterocyclus, SO₂-NH(CH₂)ₙ-Aryl, SO₂-NH(CH₂)ₙ-Heterocyclus, SO₂-N(C₁-C₆)-Alkyl)(CH₂)ₙ-Aryl, SO₂-N(C₁-C₆)-Alkyl)(CH₂)ₙ-Heterocyclus, SO₂-N((CH₂)ₙ-Aryl)₂, SO₂-N((CH₂)ₙ-(Heterocyclus)₂, NH-(CH₂)ₙ-Aryl, NH-(CH₂)ₙ-Heterocyclus, N((C₁-C₆)-Alkyl)(CH₂)ₙ-Aryl, N((C₁-C₆)-Alkyl)(CH₂)ₙ-Heterocyclus,
C(NH)(NH₂), NH₂, NH-(C₁-C₆)-Alkyl, N((C₁-C₆)-Alkyl)₂, NH-CO-(C₁-C₆)-Alkyl, NH-COO-(C₁-C₆)-Alkyl, NH-CO-Aryl, NH-CO-Heterocyclus, NH-COO-Aryl, NH-COO-Heterocyclus, NH-CO-NH-(C₁-C₆)-Alkyl, NH-CO-NH-Aryl, NH-CO-NH-Heterocyclus, Aryl, O-(CH₂)ₙ-Aryl, O-(CH₂)ₙ-Heterocyclus, wobei n = 0 - 6 sein kann, wobei der Arylrest oder Heterocyclische Rest ein bis 3-fach substituiert sein kann mit F, Cl, Br, I, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SO₂-CH₃, COOH, COO-(C₁-C₆)-Alkyl, CONH₂.

Unter einem Cycloalkylrest wird ein einen oder mehrere Ringe enthaltendes Ringssystem, welches gesättigt oder partiell ungesättigt (mit einer oder zwei Doppelbindungen) vorliegt, verstanden, das ausschließlich aus Kohlenstoffatomen aufgebaut ist, wie z.B. Cyclopropyl, Cyclopentyl, Cyclopentenyl, Cyclohexyl oder Adamantyl.

Die Cycloalkylreste können ein oder mehrfach mit geeigneten Gruppen substituiert sein, wie z.B.: F, Cl, Br, I, CF₃, OH, OCF₃, NO₂, CN, COOH, COO(C₁-C₆)Alkyl, CONH₂, CONH(C₁-C₆)Alkyl, CON[(C₁-C₆)Alkyl]₂, (C₃-C₁₀)-Cycloalkyl, (C₁-C₁₀)-Alkyl, (C₂-C₁₆)-Alkenyl, (C₂-C₆)-Alkinyl, O-(C₁-C₆)-Alkyl, CO-(C₁-C₆)-Alkyl, O-CO-(C₁-C₆)-Alkyl, O-CO-(C₁-C₆)-Aryl,
PO₃H₂, SO₃H, SO₂-NH₂, SO₂NH(C₁-C₆)-Alkyl, SO₂N[(C₁-C₆)-Alkyl]₂, S-(C₁-C₆)-Alkyl, S-(CH₂)ₙ-Aryl, S-(CH₂)ₙ-Heterocyclus, SO-(C₁-C₆)-Alkyl, SO-(CH₂)ₙ-Aryl, SO-(CH₂)ₙ-Heterocyclus, SO₂-(C₁-C₆)-Alkyl, SO₂-(CH₂)ₙ-Aryl, SO₂-(CH₂)ₙ-Heterocyclus, SO₂-NH(CH₂)ₙ-Aryl, SO₂-NH(CH₂)ₙ-Heterocyclus, SO₂-N(C₁-C₆)-Alkyl)(CH₂)ₙ-Aryl, SO₂-N(C₁-C₆)-Alkyl)(CH₂)ₙ-Heterocyclus, SO₂-N((CH₂)ₙ-Aryl)₂, SO₂-N((CH₂)ₙ-(Heterocyclus)₂, NH-(CH₂)ₙ-Aryl, NH-(CH₂)ₙ-Heterocyclus, N((C₁-C₆)-Alkyl)(CH₂)ₙ-Aryl, N((C₁-C₆)-Alkyl)(CH₂)ₙ-Heterocyclus,
C(NH)(NH₂), NH₂, NH-(C₁-C₆)-Alkyl, N((C₁-C₆)-Alkyl)₂, NH-CO-(C₁-C₆)-Alkyl, NH-COO-(C₁-C₆)-Alkyl, NH-CO-Aryl, NH-CO-Heterocyclus, NH-COO-Aryl, NH-COO-Heterocyclus, NH-CO-NH-(C₁-C₆)-Alkyl, NH-CO-NH-Aryl, NH-CO-NH-Heterocyclus, Aryl, O-(CH₂)ₙ-Aryl, O-(CH₂)ₙ-Heterocyclus, wobei n = 0 - 6 sein kann, wobei der Arylrest oder Heterocyclische Rest ein bis 3-fach substituiert sein kann mit F, Cl, Br, I, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SO₂-CH₃, COOH, COO-(C₁-C₆)-Alkyl, CONH₂.

Bicyclus ist ein teilweise ungesättigtes bicyclisches Ringsystem mit 8 bis 14 Ringgliedern, das ausschließlich Kohlenstoffatome als Ringglieder hat. Beispielhaft seien der Tetrahydronaphthyl-, alpha- oder beta-Tetralon-, Indanyl- oder Indan-1-on-ylrest genannt. Bevorzugte Bicyclusreste sind Tetrahydronaphtyl und Indanyl.

Die Bicyclusreste können ein oder mehrfach mit geeigneten Gruppen substituiert sein, wie z.B.: F, Cl, Br, I, CF₃, OH, OCF₃, NO₂, CN, COOH, COO(C₁-C₆)Alkyl, CONH₂, CONH(C₁-C₆)Alkyl, CON[(C₁-C₆)Alkyl)₂, (C₃-C₁₀)-Cycloalkyl, (C₁-C₁₀)-Alkyl, (C₂-C₆)-Alkenyl, (C₂-C₆)-Alkinyl, O-(C₁-C₆)-Alkyl, CO-(C₁-C₆)-Alkyl, O-CO-(C₁-C₆)-Alkyl, O-CO-(C₁-C₆)-Aryl,
PO₃H₂, SO₃H, SO₂-NH₂, SO₂NH(C₁-C₆)-Alkyl, SO₂N[(C₁-C₆)-Alkyl]₂, S-(C₁-C₆)-Alkyl, S-(CH₂)ₙ-Aryl, S-(CH₂)ₙ-Heterocyclus, SO-(C₁-C₆)-Alkyl, SO-(CH₂)ₙ-Aryl, SO-(CH₂)ₙ-Heterocyclus, SO₂-(C₁-C₆)-Alkyl, SO₂-(CH₂)ₙ-Aryl, SO₂-(CH₂)ₙ-Heterocyclus, SO₂-NH(CH₂)ₙ-Aryl, SO₂-NH(CH₂)ₙ-Heterocyclus, SO₂-N(C₁-C₆)-Alkyl)(CH₂)ₙ-Aryl, SO₂-N(C₁-C₆)-Alkyl)(CH₂)ₙ-Heterocyclus, SO₂-N((CH₂)ₙ-Aryl)₂, SO₂-N((CH₂)ₙ-(Heterocyclus)₂, NH-(CH₂)ₙ-Aryl, NH-(CH₂)ₙ-Heterocyclus, N((C₁-C₆)-Alkyl)(CH₂)ₙ-Aryl, N((C₁-C₆)-Alkyl)(CH₂)ₙ-Heterocyclus,
C(NH)(NH₂), NH₂, NH-(C₁-C₆)-Alkyl, N((C₁-C6)-Alkyl)₂, NH-CO-(C₁-C₆)-Alkyl, NH-COO-(C₁-C₆)-Alkyl, NH-CO-Aryl, NH-CO-Heterocyclus, NH-COO-Aryl, NH-COO-Heterocyclus, NH-CO-NH-(C₁-C₆)-Alkyl, NH-CO-NH-Aryl, NH-CO-NH-Heterocyclus, Aryl, O-(CH₂)ₙ-Aryl, O-(CH₂)ₙ-Heterocyclus, wobei n = 0 - 6 sein kann, wobei der Arylrest oder Heterocyclische Rest ein bis 3-fach substituiert sein kann mit F, Cl, Br, I, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SO₂-CH₃, COOH, COO-(C₁-C₆)-Alkyl, CONH₂.

Pharmazeutisch verträgliche Salze sind aufgrund ihrer höheren Wasserlöslichkeit gegenüber den Ausgangs- bzw. Basisverbindungen besonders geeignet für medizinische Anwendungen. Diese Salze müssen ein pharmazeutisch verträgliches Anion oder Kation aufweisen. Geeignete pharmazeutisch verträgliche Säureadditionssalze der erfindungsgemäßen Verbindungen sind Salze anorganischer Säuren, wie Salzsäure, Bromwasserstoff-, Phosphor-, Metaphosphor-, Salpeter- und Schwefelsäure sowie organischer Säuren, wie z.B. Essigsäure, Benzolsulfon-, Benzoe-, Zitronen-, Ethansulfon-, Fumar-, Glucon-, Glykol-, Isethion-, Milch-, Lactobion-, Malein-, Äpfel-, Methansulfon-, Bernstein-, p-Toluolsulfon- und Weinsäure. Geeignete pharmazeutisch verträgliche basische Salze sind Ammoniumsalze, Alkalimetallsalze (wie Natrium- und Kaliumsalze) und Erdalkalisalze (wie Magnesium- und Calciumsalze) und Salze von Trometamol (2-Amino-2-hydroxymethyl-1,3-propandiol), Diethanolamin, Lysin oder Ethylendiamin.

Salze mit einem nicht pharmazeutisch verträglichen Anion, wie zum Beispiele Trifluoracetat, gehören ebenfalls in den Rahmen der Erfindung als nützliche Zwischenprodukte für die Herstellung oder Reinigung pharmazeutisch verträglicher Salze und/oder für die Verwendung in nicht-therapeutischen, zum Beispiel in-vitro-Anwendungen.

Der hier verwendete Begriff "physiologisch funktionelles Derivat" bezeichnet jedes physiologisch verträgliche Derivat einer erfindungsgemäßen Verbindung der Formel I, z.B. einen Ester, der bei Verabreichung an einen Säuger, wie z.B. den Menschen, in der Lage ist, (direkt oder indirekt) eine Verbindung der Formel I oder einen aktiven Metaboliten hiervon zu bilden.

Zu den physiologisch funktionellen Derivaten zählen auch Prodrugs der erfindungsgemäßen Verbindungen, wie zum Beispiel in H. Okada et al., Chem. Pharm. Bull. 1994, 42, 57-61 beschrieben. Solche Prodrugs können in vivo zu einer erfindungsgemäßen Verbindung metabolisiert werden. Diese Prodrugs können selbst wirksam sein oder nicht.

Die erfindungsgemäßen Verbindungen können auch in verschiedenen polymorphen Formen vorliegen, z.B. als amorphe und kristalline polymorphe Formen. Alle polymorphen Formen der erfindungsgemäßen Verbindungen gehören in den Rahmen der Erfindung und sind ein weiterer Aspekt der Erfindung.

Nachfolgend beziehen sich alle Verweise auf "Verbindung(en) gemäß Formel I "auf Verbindung(en) der Formel I, wie vorstehend beschrieben, sowie ihre Salze, Solvate und physiologisch funktionelle Derivate wie hierin beschrieben.

### Verwendung

Die erfindungsgemäßen Verbindungen der allgemeinen Formel I besitzen eine überraschende hemmende Wirkung auf die endotheliale Lipase (EL). Das antiatheroskleotisch wirksame HDL ist das bevorzugte Substrat für EL. Eine Senkung des HDL-Spiegels führt zur Progression von Atherosklerose und ihrer Folgeerkrankungen wie Metabolisches Syndrom und Koronare Herzerkrankungen. Eine Hemmung der EL sollte somit zur Vorbeugung von atherosklerotischen Erkrankungen führen.

Die erfindungsgemäßen Verbindungen der Formel I können auch eine hemmende Wirkung auf die Triglyceridlipase aufweisen.

Weiterhin wurde gefunden, dass die hemmende Wirkung der erfindungsgemäßen Verbindungen der allgemeinen Formel I selektiv gegenüber anderen Lipasen, wie zum Beispiel der hormonsensitiven Lipase (HSL) ist.

Besonders geeignet sind solche Verbindungen zur Behandlung und/oder Prävention von
1. Störungen des Fettsäurestoffwechsels und Glucoseverwertungsstörungen
2. Störungen der Insulin-Sensitivität von Muskel-, Fett- und Leberzellen (Insulin-Resistenz) - Metabolisches Syndrom
3. Diabetes mellitus, insbesondere Typ 2 Diabetes einschließlich der Verhinderung der damit verbundenen Folgeerkrankungen.
   Besondere Aspekte sind dabei die
   - Hyperglykämie,
   - Verbesserung der Insulinresistenz,
   - Verbesserung der Glucose-Toleranz,
   - Schutz der ß-Zellen der Bauchspeicheldrüse
   - Verhinderung makro- und mikrovaskulärer Erkrankungen
4. Dyslipidämien und deren Folgen, wie z.B. Atherosklerose, koronare Herzkrankheit, zerebrovaskuläre Erkrankungen etc, insbesondere solche (aber nicht beschränkt auf), die durch einen oder mehrerer folgender Faktoren charakterisiert sind:
   - hohe Plasma-Triglycerid-, hohe postprandiale Plasma-Triglycerid-Konzentrationen
   - niedrige HDL-Cholesterin Konzentration
   - niedrige ApoA Lipoprotein-Konzentrationen
   - hohe LDL-Cholesterin Konzentrationen
   - kleine dichte LDL-Cholesterin Partikel
   - hohe ApoB Lipoprotein-Konzentrationen
5. Verschiedenen andere Zuständen, die mit dem Metabolischen Syndrom assoziert sein können, sind wie:
   - Adipositas (Fettsucht), einschließlich abdominale Adipositas
   - Thrombosen, Stadien von Hyperkoagulabilität und Thromboseneigung (arteriell und venös)
   - Hoher Blutdruck
   - Herzinsuffizienz, wie z.B. (aber nicht beschränkt auf) bei Zustand nach Myokardinfarkt, hypertensive Herzerkrankung oder Kardiomyopathie
6. weitere Krankheiten oder Zustände bei welchen zum Beispiel entzündliche Reaktionen oder die Zelldifferenzierung eine Rolle spielt sind:
   - Atherosklerose wie z.B. (aber nicht beschränkt auf) Koronarsklerose einschl. Angina pectoris oder Herzinfarkt, Hirnschlag
   - Vaskuläre Restenose oder Reverschluß
   - Chronisch entzündliche Darmerkrankungen, wie z.B. Morbus Crohn und Colitis ulcerosa
   - Pankreatitis
   - Andere entzündliche Zustände
   - Retinopathie
   - Fettzell-Tumore (adipose cell tumors)
   - Fettzell-Karzinome, wie z.B. Liposarkome
   - solide Tumoren und Neoplasien, wie z.B. (aber nicht beschränkt auf) Karzinome des Magen-Darm Traktes, der Leber, der Gallenwege und des Pankreas, endokrine Tumore, Karzinome der Lunge, der Niere und harnableitenden Organe, des Genitaltraktes, Prostata-Karzinome etc.
   - akute und chronische myeloprolifeative Erkrankungen und Lymphome
   - Angiogenese
   - Neurodegenerative Erkrankungen
   - Alzheimersche Krankheit
   - Multiple Sklerose
   - Morbus Parkinson
   - Erythemato-squamöse Dermatosen, wie z.B. Psoriasis (Schuppenflechte)
   - Akne vulgaris
   - Andere Hautkrankheiten und dermatologische Zustände, die durch PPAR moduliert werden
   - Ekzeme und Neurodermitis
   - Dermatitiden, wie z.B. seborrhoische Dermatitis oder Lichtdermatitis
   - Keratitis und Keratosen, wie z.B. seborrhoische Keratosen, senile Keratosen, aktinische Keratose, photo-induzierte Keratosen oder Keratosis follicularis
   - Keloide und Keloid-Prophylaxe
   - Warzen, einschließlich Kondylomata oder Kondylomata acuminata
   - Human papilloma viral (HPV) Infektionen, wie z.B. venerische Papillomata, virale Warzen, wie z.B. Molluscum contagiosum, Leukoplakie
   - Papulöse Dermatosen, wie z.B. Lichen planus
   - Hautkrebs, wie z.B. Basalzellkarzinome, Melanome oder kutane T-Zell Lymphome
   - Lokalisierte, benigne epidermale Tumore, wie z.B. Keratoderma, epidermale Naevi
   - Frostbeulen
   - Hoher Blutdruck
   - Syndrom X
   - Syndrom der polyzystischen Ovarien (PCOS)
   - Asthma
   - Osteoarthritis
   - Lupus erythematodes (LE) oder entzündliche rheumatische Erkrankungen, wie z.B. Rheumatoide Arthritis
   - Vaskulitis
   - Auszehrung (Kachexie)
   - Gicht
   - Ischämie/Reperfusions Syndrom
   - Akutes respiratorisches Distress Syndrom (ARDS) ("Schocklunge")

### Galenik

Die Menge einer erfindungsgemäßen Verbindung, die erforderlich ist, um den gewünschten biologischen Effekt zu erreichen, ist abhängig von einer Reihe von Faktoren, z.B. der gewählten spezifischen Verbindung, der beabsichtigten Verwendung, der Art der Verabreichung und dem klinischen Zustand des Patienten. Im allgemeinen liegt die Tagesdosis im Bereich von 0,3 mg bis 100 mg (typischerweise von 3 mg bis 50 mg) pro Tag pro Kilogramm Körpergewicht, z.B. 3 -10 mg/kg/Tag. Eine intravenöse Dosis kann z.B. im Bereich von 0,3 mg bis 1,0 mg/kg liegen, die geeigneterweise als Infusion von 10 ng bis 100 ng pro Kilogramm pro Minute verabreicht werden kann. Geeignete Infusionslösungen für diese Zwecke können z.B. von 0,1 ng bis 10 mg, typischerweise von 1 ng bis 10 mg pro Milliliter, enthalten. Einzeldosen können z.B. von 1 mg bis 10 g des Wirkstoffs enthalten. Somit können Ampullen für Injektionen beispielsweise von 1 mg bis 100 mg, und oral verabreichbare Einzeldosisformulierungen, wie zum Beispiel Tabletten oder Kapseln, können beispielsweise von 0.05 bis 1000 mg, typischerweise von 0,5 bis 600 mg enthalten. Zur Therapie der oben genannten Zustände können die Verbindungen gemäß Formel I selbst als Verbindung verwendet werden, vorzugsweise liegen sie jedoch mit einem verträglichen Träger in Form einer pharmazeutischen Zusammensetzung vor. Der Träger muss natürlich verträglich sein, in dem Sinne, dass er mit den anderen Bestandteilen der Zusammensetzung kompatibel ist und nicht gesundheitsschädlich für den Patienten ist. Der Träger kann ein Feststoff oder eine Flüssigkeit oder beides sein und wird vorzugsweise mit der Verbindung als Einzeldosis formuliert, beispielsweise als Tablette, die von 0,05% bis 95 Gew.-% des Wirkstoffs enthalten kann. Weitere pharmazeutisch aktive Substanzen können ebenfalls vorhanden sein, einschließlich weiterer erfindungsgemäßer Verbindungen. Die erfindungsgemäßen pharmazeutischen Zusammensetzungen können nach einer der bekannten pharmazeutischen Methoden hergestellt werden, die im wesentlichen darin bestehen, dass die Bestandteile mit pharmakologisch verträglichen Träger- und/oder Hilfsstoffen gemischt werden.

Erfindungsgemäße pharmazeutische Zusammensetzungen sind solche, die für orale, rektale, topische, perorale (z.B. sublinguale) und parenterale (z.B. subkutane, intramuskuläre, intradermale oder intravenöse) Verabreichung geeignet sind, wenngleich die geeignetste Verabreichungsweise in jedem Einzelfall von der Art und Schwere des zu behandelnden Zustandes und von der Art der jeweils verwendeten Verbindung gemäß Formel I abhängig ist. Auch dragierte Formulierungen und dragierte Retardformulierungen gehören in den Rahmen der Erfindung. Bevorzugt sind säure- und magensaftresistente Formulierungen. Geeignete magensaftresistente Beschichtungen umfassen Celluloseacetatphthalat, Polyvinylacetatphthalat, Hydroxypropylmethylcellulosephthalat und anionische Polymere von Methacrylsäure und Methacrylsäuremethylester.

Geeignete pharmazeutische Zubereitungen für die orale Verabreichung können in separaten Einheiten vorliegen, wie zum Beispiel Kapseln, Oblatenkapseln, Lutschtabletten oder Tabletten, die jeweils eine bestimmte Menge der Verbindung gemäß Formel I enthalten; als Pulver oder Granulate; als Lösung oder Suspension in einer wässrigen oder nicht-wässrigen Flüssigkeit; oder als eine Öl-in-Wasser- oder Wasser-in Öl-Emulsion. Diese Zusammensetzungen können, wie bereits erwähnt, nach jeder geeigneten pharmazeutischen Methode zubereitet werden, die einen Schritt umfasst, bei dem der Wirkstoff und der Träger (der aus einem oder mehreren zusätzlichen Bestandteilen bestehen kann) in Kontakt gebracht werden. Im allgemeinen werden die Zusammensetzungen durch gleichmäßiges und homogenes Vermischen des Wirkstoffs mit einem flüssigen und/oder feinverteilten festen Träger hergestellt, wonach das Produkt, falls erforderlich, geformt wird. So kann beispielsweise eine Tablette hergestellt werden, indem ein Pulver oder Granulat der Verbindung verpresst oder geformt wird, gegebenenfalls mit einem oder mehreren zusätzlichen Bestandteilen. Gepresste Tabletten können durch Tablettieren der Verbindung in frei fließender Form, wie beispielsweise einem Pulver oder Granulat, gegebenenfalls gemischt mit einem Bindemittel, Gleitmittel, inertem Verdünner und/oder einem (mehreren) oberflächenaktiven/dispergierenden Mitteln in einer geeigneten Maschine hergestellt werden. Geformte Tabletten können durch Formen der pulverförmigen, mit einem inerten flüssigen Verdünnungsmittel befeuchteten Verbindung in einer geeigneten Maschine hergestellt werden.

Pharmazeutische Zusammensetzungen, die für eine perorale (sublinguale) Verabreichung geeignet sind, umfassen Lutschtabletten, die eine Verbindung gemäß Formel I mit einem Geschmacksstoff enthalten, üblicherweise Saccharose und Gummi arabicum oder Tragant, und Pastillen, welche die Verbindung in einer inerten Basis wie Gelatine und Glycerin oder Saccharose und Gummi arabicum umfassen.

Geeignete pharmazeutische Zusammensetzungen für die parenterale Verabreichung umfassen vorzugsweise sterile wässrige Zubereitungen einer Verbindung gemäß Formel I, die vorzugsweise isotonisch mit dem Blut des vorgesehenen Empfängers sind. Diese Zubereitungen werden vorzugsweise intravenös verabreicht, wenngleich die Verabreichung auch subkutan, intramuskulär oder intradermal als Injektion erfolgen kann. Diese Zubereitungen können vorzugsweise hergestellt werden, indem die Verbindung mit Wasser gemischt wird und die erhaltene Lösung steril und mit dem Blut isotonisch gemacht wird. Injizierbare erfindungsgemäße Zusammensetzungen enthalten im allgemeinen von 0,1 bis 5 Gew.-% der aktiven Verbindung.

Geeignete pharmazeutische Zusammensetzungen für die rektale Verabreichung liegen vorzugsweise als Einzeldosis-Zäpfchen vor. Diese können hergestellt werden, indem man eine Verbindung gemäß Formel I mit einem oder mehreren herkömmlichen festen Trägern, beispielsweise Kakaobutter, mischt und das entstehende Gemisch in Form bringt.

Geeignete pharmazeutische Zusammensetzungen für die topische Anwendung auf der Haut liegen vorzugsweise als Salbe, Creme, Lotion, Paste, Spray, Aerosol oder Öl vor. Als Träger können Vaseline, Lanolin, Polyethylenglycole, Alkohole und Kombinationen von zwei oder mehreren dieser Substanzen verwendet werden. Der Wirkstoff ist im allgemeinen in einer Konzentration von 0,1 bis 15 Gew.-% der Zusammensetzung vorhanden, beispielsweise von 0,5 bis 2%.

Auch eine transdermale Verabreichung ist möglich. Geeignete pharmazeutische Zusammensetzungen für transdermale Anwendungen können als einzelne Pflaster vorliegen, die für einen langzeitigen engen Kontakt mit der Epidermis des Patienten geeignet sind. Solche Pflaster enthalten geeigneterweise den Wirkstoff in einer gegebenenfalls gepufferten wässrigen Lösung, gelöst und/oder dispergiert in einem Haftmittel oder dispergiert in einem Polymer. Eine geeignete Wirkstoff-Konzentration beträgt ca. 1% bis 35%, vorzugsweise ca. 3% bis 15%. Als eine besondere Möglichkeit kann der Wirkstoff, wie beispielsweise in Pharmaceutical Research, 2(6): 318 (1986) beschrieben, durch Elektrotransport oder lontophorese freigesetzt werden.

Die Verbindungen der Formel I zeichnen sich durch günstige Wirkungen auf Stoffwechselstörungen aus. Sie beeinflussen den Fett- und Zuckerstoffwechsel positiv, sie senken insbesondere den Triglyceridspiegel und sind zur Prävention und Behandlung von Typ II Diabetes und Arteriosklerose sowie deren vielfältigen Folgeerkrankungen geeignet.

### Kombinationen mit anderen Medikamenten

Die erfindungsgemäßen Verbindungen können alleine oder in Kombination mit einem oder mehreren weiteren pharmakologischen Wirkstoffen verabreicht werden. Insbesondere können die erfindungsgemäßen Verbindungen mit Wirkstoffen, die eine ähnliche pharmakologische Wirkung wie sie selbst aufweisen, verabreicht werden. Beispielsweise können sie in Kombination mit Wirkstoffen, die eine günstige Wirkungen auf Stoffwechselstörungen bzw. häufig damit assoziierte Erkrankungen haben, verabreicht werden. Beispiele für solche Medikamente sind
1. blutzuckersenkende Medikamente, Antidiabetika,
2. Wirkstoffe zur Behandlung von Dyslipidämien,
3. antiatherosklerotische Medikamente,
4. Mittel gegen Obesitas,
5. entzündungshemmende Wirkstoffe
6. Wirkstoffe zur Behandlung von malignen Tumoren
7. antithrombotische Wirkstoffe
8. Wirkstoffe zur Behandlung von Bluthochdruck
9. Wirkstoffe zur Behandlung von Herzinsuffizienz und
10. Wirkstoffe zur Behandlung und/oder Prävention von durch Diabetes verursachten bzw. mit Diabetes assoziierten Komplikationen.
11. Wirkstoffe zur Behandlung von neurodegenerativen Krankheiten
12. Wirkstoffe zur Behandlung von Krankheiten des Zentralen Nervensystems
13. Wirkstoffe zur Behandlung von Drogen-, Nikotin- und Alkoholabhängigkeit
14. Schmerzmittel

Sie lassen sich mit den erfindungsgemäßen Verbindungen der Formel I insbesondere zur synergistischen Wirkungsverbesserung kombinieren. Die Verabreichung der Wirkstoffkombination kann entweder durch getrennte Verabreichung der Wirkstoffe an den Patienten oder in Form von Kombinationsprodukten, bei denen mehrere Wirkstoffe in einer pharmazeutischen Zubereitung vorhanden sind, erfolgen.

Als weitere Wirkstoffe für die Kombinationspräparate sind insbesondere geeignet: Alle Antidiabetika, die in der Roten Liste 2006, Kapitel 12 genannt sind; alle Abmagerungsmittel/Appetitzügler, die in der Roten Liste 2006, Kapitel 1 genannt sind; alle Lipidsenker, die in der Roten Liste 2006, Kapitel 58 genannt sind. Sie können mit der erfindungsgemäßen Verbindung der Formel I insbesondere zur synergistischen Wirkungsverbesserung kombiniert werden. Die Verabreichung der Wirkstoffkombination kann entweder durch getrennte Gabe der Wirkstoffe an den Patienten oder in Form von Kombinationspräparaten, worin mehrere Wirkstoffe in einer pharmazeutischen Zubereitung vorliegen, erfolgen. Die meisten der nachfolgend aufgeführten Wirkstoffe sind in USP Dictionary of USAN and International Drug Names, US Pharmacopeia, Rockville 2001, offenbart.

Antidiabetika umfassen Insulin und Insulinderivate, wie z.B. Lantus^{®} (siehe www.lantus.com) oder HMR 1964 oder solche, wie sie in WO2005005477 (Novo Nordisk) beschrieben sind, schnell wirkende Insuline (siehe US 6,221,633), inhalierbare Insuline, wie z. B. Exubera ^{®} oder orale Insuline, wie z. B. IN-105 (Nobex) oder Oral-lyn™ (Generex Biotechnology), GLP-1-Derivate wie z.B. Exenatide, Liraglutide oder diejenigen die in WO 98/08871 oder WO2005027978 von Novo Nordisk A/S, in WO 01/04156 von Zealand oder in WO 00/34331 von Beaufour-Ipsen offenbart wurden, Pramlintide Acetat (Symlin; Amylin Pharmaceuticals), sowie oral wirksame hypoglykämische Wirkstoffe.

Die Wirkstoffe umfassen vorzugsweise
Sulfonylharnstoffe,
Biguanide,
Meglitinide,
Oxadiazolidindione,
Thiazolidindione,
Glukosidase-Inhibitoren,
Hemmstoffe der Glykogen Phosphorylase,
Glukagon-Antagonisten,
Glukokinase Aktivatoren,
Inhibitoren der Fructose-1,6-bisphosphatase,
Modulatoren des Glukosetransporters-4 (GLUT4),
Inhibitoren der Glutamin-Fructose-6-Phosphat-Amidotransferase (GFAT), GLP-1-Agonisten,
Kaliumkanalöffner, wie z.B. diejenigen, die in WO 97/26265 und WO 99/03861 von Novo Nordisk A/S offenbart wurden,
Inhibitoren der Dipeptidylpeptidase-IV (DPP-IV),
Insulin-Sensitizer,
Inhibitoren von Leberenzymen, die an der Stimulation der Glukoneogenese und/oder Glykogenolyse beteiligt sind,
Modulatoren der Glukoseaufnahme, des Glukosetransports und der Glukoserückresorption,
Hemmstoffe der 11ß-HSD1,
Inhibitoren der Protein-Tyrosin-Phosphatase-1B (PTP1 B),
Modulatoren des natrium-abhängigen Glukosetransporters 1 oder 2 (SGLT1, SGLT2), den Fettstoffwechsel verändernde Verbindungen wie antihyperlipidämische Wirkstoffe und antilipidämische Wirkstoffe,
Verbindungen, die die Nahrungsmitteleinnahme verringern,
Verbindungen, die die Thermogenese erhöhen,
PPAR- und RXR-Modulatoren und
Wirkstoffe, die auf den ATP-abhängigen Kaliumkanal der Betazellen wirken.

Bei einer Ausführungsform der Erfindung wird die Verbindung der Formel I in Kombination mit einem HMGCoA-Reduktase Inhibitor, wie Simvastatin, Fluvastatin, Pravastatin, Lovastatin, Atorvastatin, Cerivastatin, Rosuvastatin oder L-659699, verabreicht.

Bei einer Ausführungsform der Erfindung wird die Verbindung der Formel I in Kombination mit einem Cholesterinresorptionsinhibitor, wie z.B. Ezetimibe, Tiqueside, Pamaqueside, FM-VP4 (sitostanol/campesterol ascorbyl phosphat; Forbes Medi-Tech, WO2005042692), MD-0727 (Microbia Inc., WO2005021497) oder mit Verbindungen, wie in WO2002066464 (Kotobuki Pharmaceutical Co. Ltd.), WO2005062824 (Merck, & Co.) oder WO2005061451 und WO2005061452 (AstraZeneca AB) beschrieben, verabreicht.

Bei einer Ausführungsform der Erfindung wird die Verbindung der Formel I in Kombination mit einem PPAR gamma Agonisten, wie z.B. Rosiglitazon, Pioglitazon, JTT-501, GI 262570, R-483 oder CS-011 (Rivoglitazon), verabreicht.

Bei einer Ausführungsform der Erfindung wird die Verbindung der Formel I in Kombination mit einem PPAR alpha Agonisten, wie z.B. GW9578, GW-590735, K-111, LY-674, KRP-101 oder DRF-10945, verabreicht.

Bei einer Ausführungsform der Erfindung wird die Verbindung der Formel I in Kombination mit einem gemischten PPAR alpha/gamma Agonisten, wie z.B. Muraglitazar, Tesaglitazar, Naveglitazar, LY-510929, ONO-5129, E-3030 oder wie in WO00/64888, WO00/64876, WO03/020269, WO2004075891, WO2004076402, WO2004075815, WO2004076447, WO2004076428, WO2004076401, WO2004076426, WO2004076427, WO2006018118, WO2006018115, and WO2006018116 oder in J.P. Berger et al., TRENDS in Pharmacological Sciences 28(5), 244-251, 2005 beschrieben, verabreicht.

Bei einer Ausführungsform der Erfindung wird die Verbindung der Formel I in Kombination mit einem PPAR delta Agonisten, wie z.B. GW-501516, oder wie in WO2005097762, WO2005097786, WO2005097763, WO2006029699 beschrieben verabreicht.

Bei einer Ausführungsform der Erfindung wird die Verbindung der Formel I in Kombination mit Metaglidasen oder mit MBX-2044 oder anderen partiellen PPAR gamma Agonisten/Antagonisten verabreicht.

Bei einer Ausführungsform der Erfindung wird die Verbindung der Formel I in Kombination mit einem Fibrat, wie z.B. Fenofibrat, Clofibrat oder Bezafibrat, verabreicht.

Bei einer Ausführungsform der Erfindung wird die Verbindung der Formel I in Kombination mit einem MTP-Inhibitor, wie z.B. Implitapide, BMS-201038, R-103757 oder solchen wie in WO2005085226 beschrieben, verabreicht.

Bei einer Ausführungsform der Erfindung wird die Verbindung der Formel I in Kombination mit einem CETP-Inhibitor, wie z.B. Torcetrapib oder JTT-705, verabreicht.

Bei einer Ausführungsform der Erfindung wird die Verbindung der Formel I in Kombination mit einem Gallensäureresorptionsinhibitor (siehe z.B. US 6,245,744, US 6,221,897 oder WO00/61568), wie z.B. HMR 1741 oder solchen wie in DE 10 2005 033099.1 und DE 10 2005 033100.9 beschrieben, verabreicht.

Bei einer Ausführungsform der Erfindung wird die Verbindung der Formel I in Kombination mit einem polymeren Gallensäureadsorber, wie z.B. Cholestyramin oder Colesevelam, verabreicht.

Bei einer Ausführungsform der Erfindung wird die Verbindung der Formel I in Kombination mit einem LDL-Rezeptorinducer (siehe US 6,342,512), wie z.B. HMR1171, HMR1586 oder solchen wie in WO2005097738 beschrieben, verabreicht.

Bei einer Ausführungsform wird die Verbindung der Formel I in Kombination mit Omacort® (Omega-3-Fettsäuren; hochkonzentrierte Ethylester der Eicosapentaensäure und der Docosahexaensäure) verabreicht.

Bei einer Ausführungsform der Erfindung wird die Verbindung der Formel I in Kombination mit einem ACAT-Inhibitor, wie z.B. Avasimibe, verabreicht.

Bei einer Ausführungsform der Erfindung wird die Verbindung der Formel I in Kombination mit einem Antioxidans, wie z.B. OPC-14117, Probucol, Tocopherol, Ascorbinsäure, ß-Caroten oder Selen, verabreicht.

Bei einer Ausführungsform der Erfindung wird die Verbindung der Formel I in Kombination mit einem Vitamin, wie z. B. Vitamin B6 oder Vitamin B12, verabreicht.

Bei einer Ausführungsform der Erfindung wird die Verbindung der Formel I in Kombination mit einem Lipoprotein-Lipase Modulator, wie z.B. Ibrolipim (NO-1886), verabreicht.

Bei einer Ausführungsform der Erfindung wird die Verbindung der Formel I in Kombination mit einem ATP-Citrat-Lyase Inhibitor, wie z.B. SB-204990, verabreicht.

Bei einer Ausführungsform der Erfindung wird die Verbindung der Formel I in Kombination mit einem Squalen Synthetase Inhibitor, wie z.B. BMS-188494 oder wie in WO2005077907 beschrieben, verabreicht.

Bei einer Ausführungsform der Erfindung wird die Verbindung der Formel I in Kombination mit einem Lipoprotein(a) Antagonist, wie z.B. Gemcabene (CI-1027), verabreicht.

Bei einer Ausführungsform der Erfindung wird die Verbindung der Formel I in Kombination mit einem HM74A Rezeptor Agonisten, wie z.B. Nicotinsäure, verabreicht.

Bei einer Ausführungsform der Erfindung wird die Verbindung der Formel I in Kombination mit einem Lipase Inhibitor, wie z.B. Orlistat oder Cetilistat (ATL-962), verabreicht.

Bei einer Ausführungsform der Erfindung wird die Verbindung der Formel I in Kombination mit Insulin verabreicht.

Bei einer Ausführungsform der Erfindung wird die Verbindung der Formel I in Kombination mit einem Sulfonylharnstoff, wie z.B. Tolbutamid, Glibenclamid, Glipizid oder Glimepirid, verabreicht.

Bei einer Ausführungsform der Erfindung wird die Verbindung der Formel I in Kombination mit einem Biguanid, wie z.B. Metformin, verabreicht.

Bei wieder einer Ausführungsform der Erfindung wird die Verbindung der Formel I in Kombination mit einem Meglitinid, wie z.B. Repaglinide oder Nateglinid, verabreicht.

Bei einer Ausführungsform der Erfindung wird die Verbindung der Formel I in Kombination mit einem Thiazolidindion, wie z.B. Troglitazon, Ciglitazon, Pioglitazon, Rosiglitazon oder den in WO 97/41097 von Dr. Reddy's Research Foundation offenbarten Verbindungen, insbesondere 5-[[4-[(3,4-Dihydro-3-methyl-4-oxo-2-chinazolinylmethoxy]phenyl]methyl]-2,4-thiazolidindion, verabreicht.

Bei einer Ausführungsform der Erfindung wird die Verbindung der Formel I in Kombination mit einem α-Glukosidase-Inhibitor, wie z.B. Miglitol oder Acarbose, verabreicht.

Bei einer Ausführungsform der Erfindung wird die Verbindung der Formel I in Kombination mit einem Wirkstoff verabreicht, der auf den ATP-abhängigen Kaliumkanal der Betazellen wirkt, wie z.B. Tolbutamid, Glibenclamid, Glipizid, Glimepirid oder Repaglinid.

Bei einer Ausführungsform der Erfindung wird die Verbindung der Formel I in Kombination mit mehr als einer der vorstehend genannten Verbindungen, z.B. in Kombination mit einem Sulfonylharnstoff und Metformin, einem Sulfonylharnstoff und Acarbose, Repaglinid und Metformin, Insulin und einem Sulfonylharnstoff, Insulin und Metformin, Insulin und Troglitazon, Insulin und Lovastatin, etc., verabreicht.

Bei einer Ausführungsform der Erfindung wird die Verbindung der Formel I in Kombination mit einem Hemmstoff der Glykogen Phosphorylase, wie z.B. PSN-357 oder FR-258900 oder solchen wie in WO2003084922, WO2004007455, WO2005073229-31 oder WO2005067932 beschrieben, verabreicht.

Bei einer Ausführungsform der Erfindung wird die Verbindung der Formel I in Kombination mit Glukagon-Rezeptor-Antagonisten, wie z.B. A-770077, NNC-25-2504 oder wie in WO2004100875 oder WO2005065680 beschrieben, verabreicht.

Bei einer Ausführungsform der Erfindung wird die Verbindung der Formel I in Kombination mit Aktivatoren der Glukokinase, wie z. B. RO-4389620, LY-2121260 (WO2004063179), PSN-105, PSN-110, GKA-50oder, solchen wie sie z. B. von Prosidion in WO2004072031, WO2004072066, WO 05103021 oder WO 06016178, von Roche in WO 00058293, WO 00183465, WO 00183478, WO 00185706, WO 00185707, WO 01044216, GB 02385328, WO 02008209, WO 02014312, WO 0246173, WO 0248106, DE 10259786, WO 03095438, US 04067939 oder WO 04052869, von Novo Nordisk in EP 1532980, WO 03055482, WO 04002481, WO 05049019, WO 05066145 oder WO 05123132, von Merck/Banyu in WO 03080585, WO03097824, WO 04081001, WO 05063738 oder WO 05090332, von Eli Lilly in WO 04063194, oder von Astra Zeneca in WO 01020327, WO 03000262, WO 03000267, WO 03015774, WO 04045614, WO 04046139, WO 05044801, WO 05054200, WO 05054233, WO 05056530, WO 05080359, WO 05080360 oder WO 05121110 beschrieben sind, verabreicht.

Bei einer Ausführungsform der Erfindung wird die Verbindung der Formel I in Kombination mit einem Inhibitor der Glukoneogenese, wie z. B. FR-225654, verabreicht.

Bei einer Ausführungsform der Erfindung wird die Verbindung der Formel I in Kombination mit Inhibitoren der Fructose-1,6-bisphosphatase (FBPase), wie z.B. CS-917, verabreicht.

Bei einer Ausführungsform der Erfindung wird die Verbindung der Formel I in Kombination mit Modulatoren des Glukosetransporters-4 (GLUT4), wie z.B. KST-48 (D.-O. Lee et al.: Arzneim.-Forsch. Drug Res. 54 (12), 835 (2004)), verabreicht.

Bei einer Ausführungsform der Erfindung wird die Verbindung der Formel I in Kombination mit Inhibitoren der Glutamin-Fructose-6-Phosphat-Amidotransferase (GFAT), wie sie z. B. in WO2004101528 beschrieben sind, verabreicht.

Bei einer Ausführungsform der Erfindung wird die Verbindung der Formel I in Kombination mit Inhibitoren der Dipeptidylpeptidase-IV (DPP-IV), wie z. B. Vildagliptin (LAF-237), Sitagliptin (MK-0431), Saxagliptin ((BMS-477118), GSK-823093, PSN-9301, SYR-322, SYR-619, TA-6666, TS-021, GRC-8200, GW-825964X oder wie sie in WO2003074500, WO2003106456, WO200450658, WO2005058901, WO2005012312, WO2005/012308, PCT/EP2005/007821, PCT/EP2005/008005, PCT/EP2005/008002, PCT/EP2005/008004, PCT/EP2005/008283, DE 102005012874.2 oder DE 10 2005 012873.4 beschrieben sind, verabreicht..

Bei einer Ausführungsform der Erfindung wird die Verbindung der Formel I in Kombination mit Hemmstoffen der 11-beta-Hydroxysteroid-Dehydrogenase-1(11ß-HSD1), wie z. B. BVT-2733 oder solche, wie sie z. B. in WO200190090-94, WO200343999, WO2004112782, WO200344000, WO200344009, WO2004112779, WO2004113310, WO2004103980, WO2004112784, WO2003065983, WO2003104207, WO2003104208, WO2004106294, WO2004011410, WO2004033427, WO2004041264, WO2004037251, WO2004056744, WO2004065351, WO2004089367, WO2004089380, WO200408947071, WO2004089896, WO2005016877 oder WO2005097759 beschrieben sind, verabreicht.

Bei einer Ausführungsform der Erfindung wird die Verbindung der Formel I in Kombination mit Inhibitoren der Protein-Tyrosin-Phosphatase-1B (PTP1B), wie sie z. B. in WO200119830-31, WO200117516, WO2004506446, WO2005012295, PCT/EP2005/005311, PCT/EP2005/005321, PCT/EP2005/007151, PCT/EP2005/01294 oder DE 10 2004 060542.4 beschrieben sind, verabreicht.

Bei einer Ausführungsform der Erfindung wird die Verbindung der Formel I in Kombination mit Modulatoren des natrium-abhängigen Glukosetransporters 1 oder 2 (SGLT1, SGLT2), wie z.B. KGA-2727, T-1095 und SGL-0010 oder wie sie z. B. in WO2004007517, WO200452903, WO200452902, WO2005121161, WO2005085237, JP2004359630 oder von A. L. Handlon in Expert Opin. Ther. Patents (2005) 15(11), 1531-1540 beschrieben sind, verabreicht.

Bei einer Ausführungsform der Erfindung wird die Verbindung der Formel I in Kombination mit Inhibitoren der hormon-sensitiven Lipase (HSL), wie z. B. in in WO01/17981, WO01/66531, WO2004035550, WO2005073199 oder WO03/051842 beschrieben, verabreicht.

Bei einer Ausführungsform der Erfindung wird die Verbindung der Formel I in Kombination mit Hemmstoffen der Acetyl-CoA Carboxylase (ACC) wie z. B. solchen wie in WO199946262, WO200372197, WO2003072197 oder WO2005044814 beschrieben, verabreicht.

Bei einer Ausführungsform der Erfindung wird die Verbindung der Formel I in Kombination mit einem Inhibitor der Phosphoenolpyruvatcarboxykinase (PEPCK), wie z.B. solchen, wie in WO2004074288 beschrieben, verabreicht.

Bei einer Ausführungsform der Erfindung wird die Verbindung der Formel I in Kombination mit einem Inhibitor der Glykogen Synthase Kinase-3 beta (GSK-3 beta), wie z. B. in US2005222220, WO2004046117, WO2005085230, WO2005111018, WO2003078403, WO2004022544, WO2003106410, WO2005058908. US2005038023, WO2005009997, US2005026984, WO2005000836, WO2004106343, EP1460075, WO2004014910, WO2003076442, WO2005087727 oder WO2004046117 beschrieben, verabreicht.

Bei einer Ausführungsform der Erfindung wird die Verbindung der Formel I in Kombination mit einem Inhibitor der Protein Kinase C beta (PKC beta), wie z. B. Ruboxistaurin, verabreicht.

Bei einer Ausführungsform der Erfindung wird die Verbindung der Formel I in Kombination mit einem Endothelin-A-Rezeptor Antagonisten, wie z. B. Avosentan (SPP-301), verabreicht.

Bei einer Ausführungsform der Erfindung wird die Verbindung der Formel I in Kombination mit Inhibitoren der "I-kappaB Kinase" (IKK Inhibitoren), wie sie z. B. in WO2001000610, WO2001030774, WO2004022553 oder WO2005097129 beschrieben sind, verabreicht.

Bei einer Ausführungsform der Erfindung wird die Verbindung der Formel I in Kombination mit Modulatoren des Glukocorticoidrezeptors, wie sie z. B. in WO2005090336 beschrieben sind, verabreicht.

Bei einer weiteren Ausführungsform der Erfindung wird die Verbindung der Formel I in Kombination mit CART-Modulatoren (siehe "Cocaine-amphetamine-regulated transcript influences energy metabolism, anxiety and gastric emptying in mice" Asakawa, A. et al.: Hormone and Metabolic Research (2001), 33(9), 554-558); NPY-Antagonisten wie z.B. Naphthalin-1-sulfonsäure-{4-[(4-amino-quinazolin-2-ylamino)-methyl]-cyclohexylmethyl}-amid Hydrochlorid (CGP 71683A);
Peptid YY 3-36 (PYY3-36) oder analoge Verbindungen, wie z. B. CJC-1682 (PYY3-36 konjugiert mit humanem Serum Albumin über Cys34), CJC-1643 (Derivat des PYY3-36, welches sich in vivo an Serum Albumin konjugiert) oder solche, wie sie in WO2005080424 beschrieben sind;
Cannabinoid Rezeptor 1 Antagonisten, wie z.B. Rimonabant, SR147778 oder solche wie sie in z. B. EP 0656354, WO 00/15609, WO 02/076949, WO2005080345, WO2005080328, WO2005080343, WO2005075450, WO2005080357, WO200170700, WO2003026647-48, WO200302776, WO2003040107, WO2003007887, WO2003027069, US6,509,367, WO200132663, WO2003086288, WO2003087037, WO2004048317, WO2004058145, WO200308493 WO2003084943, WO2004058744, WO2004013120, WO2004029204, WO2004035566, WO2004058249, WO2004058255, WO2004058727, WO2004069838, US20040214837, US20040214855, US20040214856, WO2004096209, WO2004096763, WO2004096794, WO2005000809, WO2004099157, US20040266845, WO2004110453, WO2004108728, WO2004000817, WO2005000820, US20050009870, WO200500974, WO200411103334, WO200411038-39, WO2005016286, WO2005007111, WO2005007628, US20050054679, WO2005027837, WO2005028456, WO200506376162, WO2005061509 oder WO2005077897 beschrieben sind;
MC4-Agonisten (z.B. 1-Amino-1,2,3,4-tetrahydro-naphthalin-2-carbonsäure [2-(3a-benzyl-2-methyl-3-oxo-2,3,3a,4,6,7-hexahydro-pyrazolo[4,3-c]pyridin-5-yl)-1-(4-chloro-phenyl)-2-oxo-ethyl]-amid; (WO 01/91752)) oder LB53280, LB53279, LB53278 oder THIQ, MB243, RY764, CHIR-785, PT-141 oder solche wie sie in WO2005060985, WO2005009950, WO2004087159, WO2004078717, WO2004078716, WO2004024720, US20050124652, WO2005051391, WO2004112793, WOUS20050222014, US20050176728, US20050164914, US20050124636, US20050130988, US20040167201, WO2004005324, WO2004037797, WO2005042516, WO2005040109, WO2005030797, US20040224901, WO200501921, WO200509184, WO2005000339, EP1460069, WO2005047253, WO2005047251, EP1538159, WO2004072076, WO2004072077 oder WO2006024390 beschrieben sind;
Orexin-RezeptorAntagonisten (z.B. 1-(2-Methyl-benzoxazol-6-yl)-3-[1,5]naphthyridin-4-yl-harnstoff Hydrochlorid (SB-334867-A) oder solche, wie sie z. B. in WO200196302, WO200185693, WO2004085403 oder WO2005075458 beschrieben sind);
Histamin H3 Rezeptor Agonisten (z. B. 3-Cyclohexyl-1-(4,4-dimethyl-1,4,6,7-tetrahydro-imidazo[4,5-c]pyridin-5-yl)-propan-1-on Oxalsäuresalz (WO 00/63208) oder solche, wie sie in WO200064884, WO2005082893 beschrieben sind);
CRF-Antagonisten (z.B. [2-Methyl-9-(2,4,6-trimethyl-phenyl)-9H-1,3,9-triaza-fluoren-4-yl]-dipropyl-amin (WO 00/66585));
CRF BP-Antagonisten (z.B. Urocortin);
Urocortin-Agonisten;
β3-Agonisten (wie z.B. 1-(4-Chloro-3-methanesulfonylmethyl-phenyl)-2-[2-(2,3-dimethyl-1 H-indol-6-yloxy)-ethylamino]-ethanol Hydrochlorid (WO 01/83451));
MSH (Melanocyt-stimulierendes Hormon)-Agonisten;
MCH (melanin-konzentrierendes Hormon) Rezeptor Antagonisten (wie z. B. NBI-845, A-761, A-665798, A-798, ATC-0175, T-226296, T-71, GW-803430 oder solche Verbindungen, wie sie in WO2003/15769, WO2005085200, WO2005019240, WO2004011438, WO2004012648, WO2003015769, WO2004072025, WO2005070898, WO2005070925, WO2006018280, WO2006018279, WO2004039780, WO2003033476, WO2002006245, WO2002002744, WO2003004027 oder FR2868780 beschrieben sind);
CCK-A Agonisten (wie z.B. {2-[4-(4-Chloro-2,5-dimethoxy-phenyl)-5-(2-cyclohexyl-ethyl)-thiazol-2-ylcarbamoyl]-5,7-dimethyl-indol-1-yl}-essigsäure Trifluoressigsäuresalz (WO 99/15525), SR-146131 (WO 0244150) oder SSR-125180); Serotonin-Wiederaufnahme-Inhibitoren (z.B. Dexfenfluramine);
gemischte Serotonin- und noradrenerge Verbindungen (z.B. WO 00/71549); 5-HT-Rezeptor Agonisten z.B. 1-(3-Ethyl-benzofuran-7-yl)-piperazin Oxalsäuresalz (WO 01/09111);
5-HT2C Rezeptor Agonisten (wie z.B. APD-356, BVT-933 oder solche, wie sie in WO200077010, WO20077001-02, WO2005019180, WO2003064423, WO200242304 oder WO2005082859 beschrieben sind);
5-HT6 Rezeptor Antagonisten, wie sie z.B. in WO2005058858 beschrieben sind; Bombesin-Rezeptor Agonisten (BRS-3 Agonisten);
Galanin-Rezeptor Antagonisten;
Wachstumshormon (z.B. humanes Wachstumshormon oder AOD-9604);
Wachstumshormon freisetzende Verbindungen (6-Benzyloxy-1-(2-diisopropylamino-ethylcarbamoyl)-3,4-dihydro-1H-isochinolin-2-carbonsäuretertiärbutylester (WO 01/85695));
Growth Hormone Secretagogue Receptor Antagonisten (Ghrelin Antagonisten) wie z. B. A-778193 oder solche, wie sie in WO2005030734 beschrieben sind; TRH-Agonisten (siehe z.B. EP 0 462 884);
entkoppelnde Protein 2- oder 3-Modulatoren;
Leptinagonisten (siehe z.B. Lee, Daniel W.; Leinung, Matthew C.; Rozhavskaya-Arena, Marina; Grasso, Patricia. Leptin agonists as a potential approach to the treatment of obesity. Drugs of the Future (2001), 26(9), 873-881);
DA-Agonisten (Bromocriptin oder Doprexin);
Lipase/Amylase-Inhibitoren (wie sie z.B. in WO 00/40569 beschrieben sind); Inhibitoren der Diacylglycerol O-Acyltransferasen (DGATs) wie z. B. in US2004/0224997, WO2004094618, WO200058491, WO2005044250, WO2005072740, JP2005206492 oder WO2005013907 beschrieben;
Inhibitoren der Fettsäuresynthase (FAS) wie z.B. C75 oder solche, wie in WO2004005277 beschrieben;
Oxyntomodulin;
Oleoyl-Estron
oder Agonisten des Schilddrüsenhormonrezeptors (thyroid hormone receptor agonists) wie z. B: KB-2115 oder solche, wie in WO20058279, WO200172692, WO200194293, WO2003084915, WO2004018421 oder WO2005092316 beschrieben, verabreicht.

Bei einer Ausführungsform der Erfindung ist der weitere Wirkstoff Leptin;
siehe z.B. "Perspectives in the therapeutic use of leptin", Salvador, Javier; Gomez-Ambrosi, Javier; Fruhbeck, Gema, Expert Opinion on Pharmacotherapy (2001), 2(10), 1615-1622.

Bei einer Ausführungsform der Erfindung ist der weitere Wirkstoff Dexamphetamin oder Amphetamin.

Bei einer Ausführungsform der Erfindung ist der weitere Wirkstoff Fenfluramin oder Dexfenfluramin.

Bei noch einer Ausführungsform der Erfindung ist der weitere Wirkstoff Sibutramin.

Bei einer Ausführungsform der Erfindung ist der weitere Wirkstoff Mazindol oder Phentermin.

Bei einer Ausführungsform der Erfindung wird die Verbindung der Formel I in Kombination mit Ballaststoffen, vorzugsweise unlöslichen Ballaststoffen (siehe z.B. Carob/ Caromax^{®} (Zunft H J; et al., Carob pulp preparation for treatment of hypercholesterolemia, ADVANCES IN THERAPY (2001 Sep-Oct), 18(5), 230-6). Caromax ist ein Carob enthaltendes Produkt der Fa. Nutrinova, Nutrition Specialties & Food Ingredients GmbH, Industriepark Höchst, 65926 Frankfurt / Main) verabreicht. Die Kombination mit Caromax^{®} kann in einer Zubereitung erfolgen oder durch getrennte Gabe von Verbindungen der Formel I und Caromax^{®}. Caromax^{®} kann dabei auch in Form von Lebensmitteln, wie z.B. in Backwaren oder Müsliriegeln, verabreicht werden.

Bei einer Ausführungsform der Erfindung wird die Verbindung der Formel I in Kombination mit PDE Inhibitoren (Phosphodiesterase), wie sie z. B. in WO2003/077949 oder WO2005012485 beschrieben sind, verabreicht.

Bei einer Ausführungsform der Erfindung wird die Verbindung der Formel I in Kombination mit NAR-1 (Nicotinic Acid Receptor) Agonisten, wie sie z. B. in WO2004094429 beschrieben sind, verabreicht.

Bei einer Ausführungsform der Erfindung wird die Verbindung der Formel I in Kombination mit CB2 (Cannabinoid Receptor) Agonisten, wie sie z. B. in US2005/143448 beschrieben sind, verabreicht.

Bei einer Ausführungsform der Erfindung wird die Verbindung der Formel I in Kombination mit Histamine-1-Agonisten, wie sie z. B. in WO2005101979 beschrieben sind, verabreicht.

Bei einer Ausführungsform der Erfindung wird die Verbindung der Formel I in Kombination mit Bupropion, wie in WO2006017504 beschrieben, verabreicht.

Bei einer Ausführungsform der Erfindung wird die Verbindung der Formel I in Kombination mit Opioid Antagonisten, wie sie z. B. in WO2005107806 oder WO2004094429 beschrieben sind, verabreicht.

Bei einer Ausführungsform der Erfindung wird die Verbindung der Formel I in Kombination mit Neutralen Endopeptidase Inhibitoren , wie sie z. B. in WO200202513, WO2002/06492, WO 2002040008, WO2002040022 oder WO2002047670 beschrieben sind, verabreicht.

Bei einer Ausführungsform der Erfindung wird die Verbindung der Formel I in Kombination mit NPY Inhibitoren (Neuropeptid Y), wie sie z. B. in WO2002047670 beschrieben sind, verabreicht.

Bei einer Ausführungsform der Erfindung wird die Verbindung der Formel I in Kombination mit Natrium/Wasserstoff Austausch Inhibitoren, wie sie z. B. in WO2003092694 beschrieben sind, verabreicht.

Bei einer Ausführungsform der Erfindung wird die Verbindung der Formel I in Kombination mit Modulatoren des Glukocorticoidrezeptors, wie sie z. B. in WO2005090336 beschrieben sind, verabreicht.

Bei einer Ausführungsform der Erfindung wird die Verbindung der Formel I in Kombination mit Nikotin Rezeptor Agonisten, wie sie z. B. in WO2004094429 beschrieben sind, verabreicht.

Bei einer Ausführungsform der Erfindung wird die Verbindung der Formel I in Kombination mit NRIs (Norepinephrine reuptake inhibitors), wie sie z. B. in WO2002053140 beschrieben sind, verabreicht.

Bei einer Ausführungsform der Erfindung wird die Verbindung der Formel I in Kombination mit MOA (E-beta-methoxyacrylate), wie z.B. Segeline oder wie sie z. B. in WO2002053140 beschrieben sind, verabreicht.

Bei einer Ausführungsform der Erfindung wird die Verbindung der Formel I in Kombination mit antithrombotischen Wirkstoffen, wie z. B. Clopidrogel, verabreicht.

Es versteht sich, dass jede geeignete Kombination der erfindungsgemäßen Verbindungen mit einer oder mehreren der vorstehend genannten Verbindungen und wahlweise einer oder mehreren weiteren pharmakologisch wirksamen Substanzen als unter den Schutzbereich der vorliegenden Erfindung fallend angesehen wird.

Zu den vorstehend genannten Entwicklungskodes werden nachfolgend teilweise die Formeln aufgeführt.

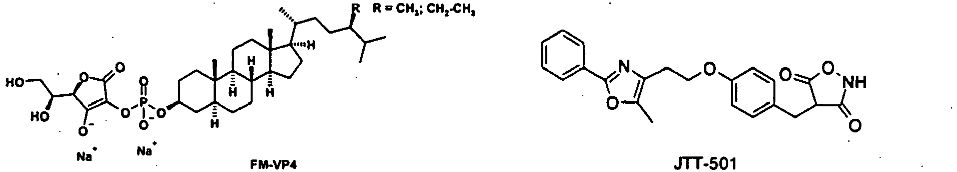

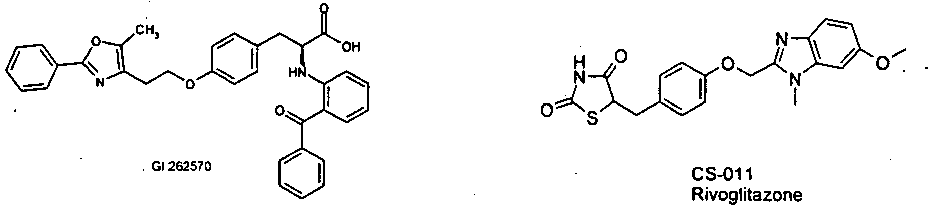

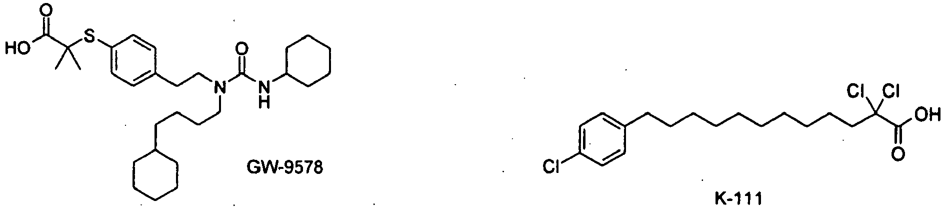

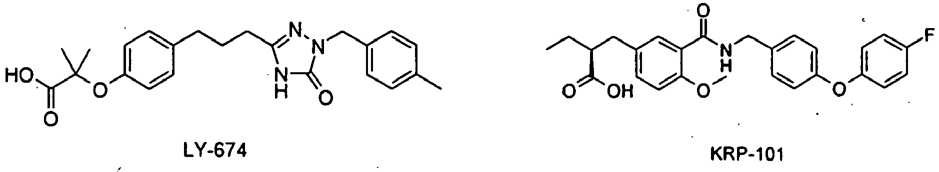

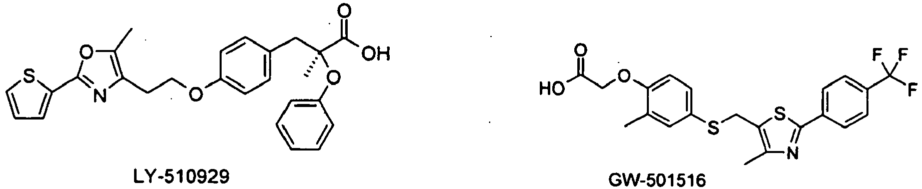

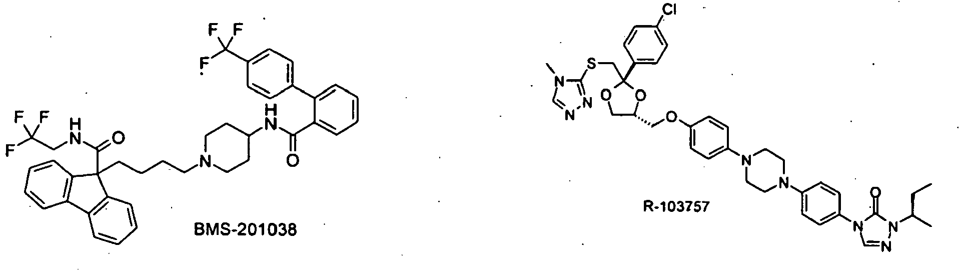

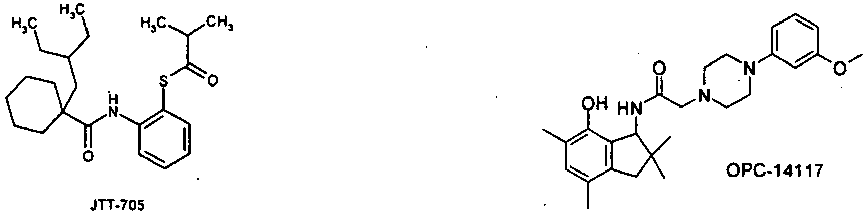

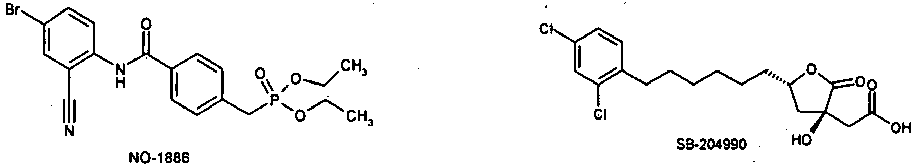

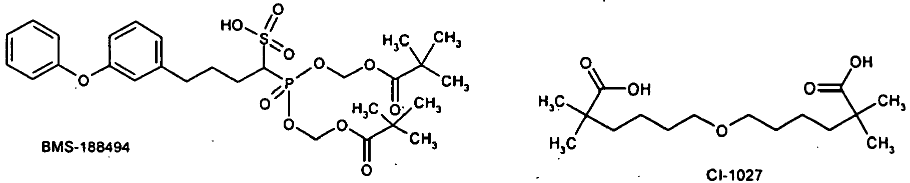

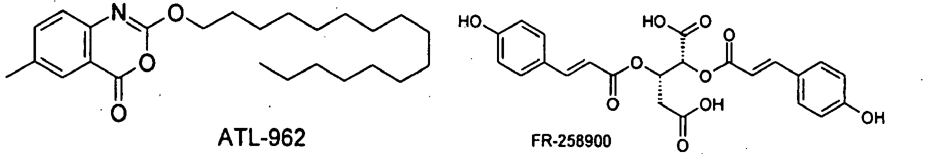

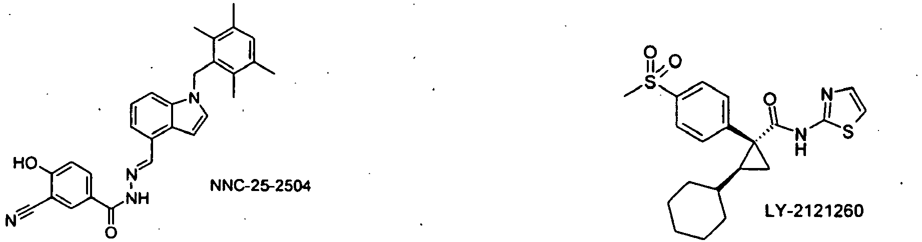

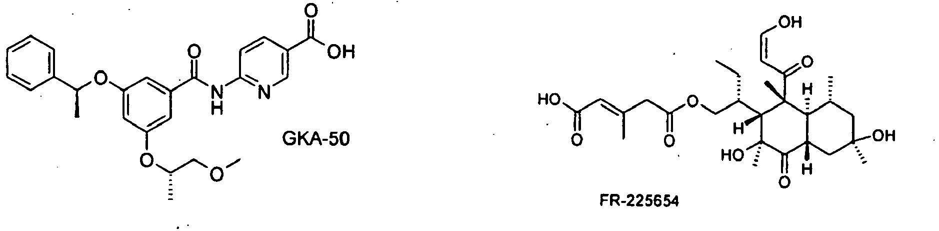

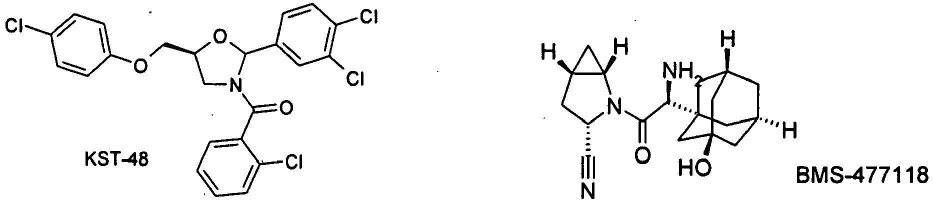

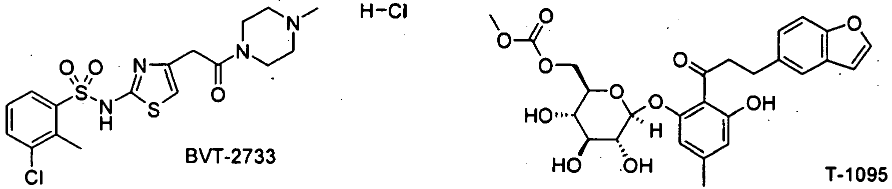

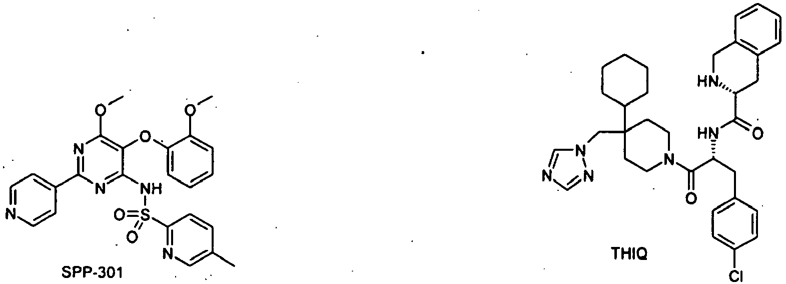

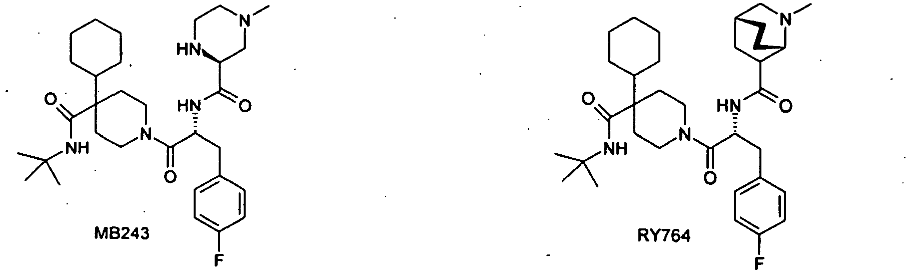

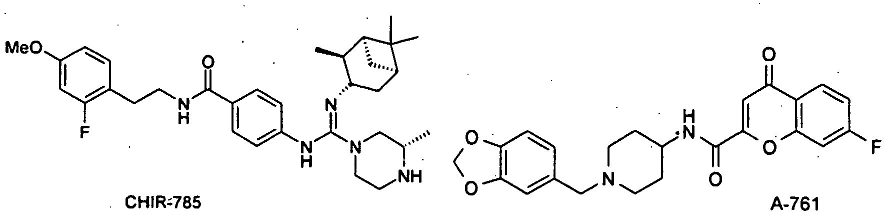

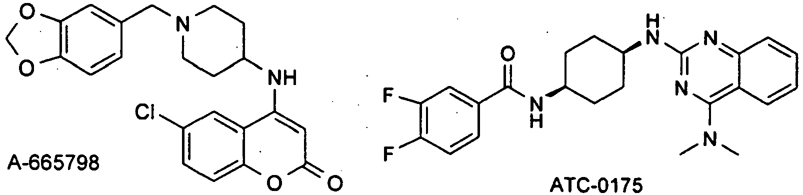

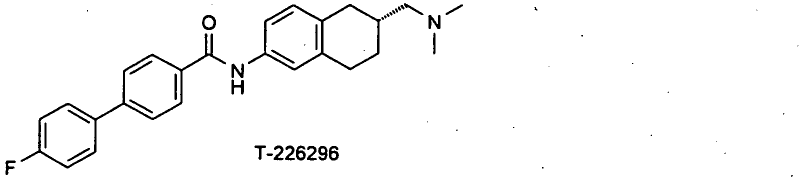

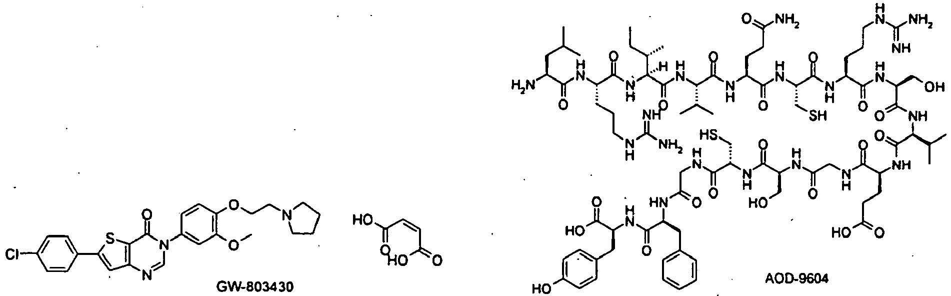

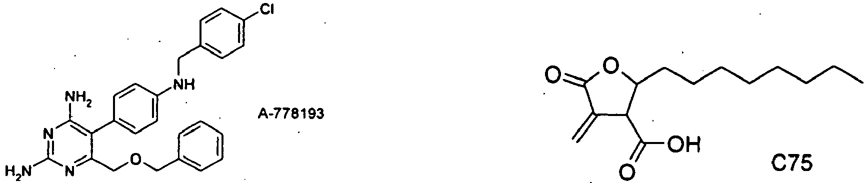

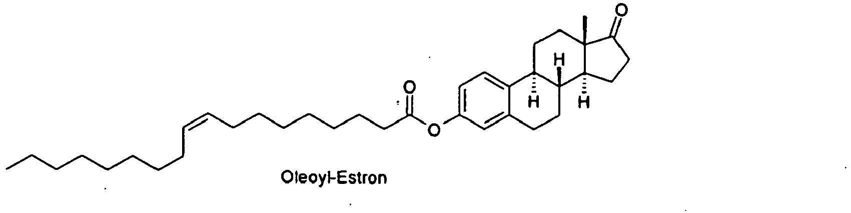

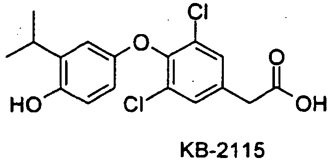

Die Wirksamkeit der erfindungsgemäßen Verbindungen der Formel I wurde an folgendem Enzymtestsystem geprüft:

### Test auf Hemmung der EL:

Endotheliale Lipase wird als sekretorisches Protein von rekombinanten Zell-Linien (CHO, HEK293) in hoher Konzentration in Zellkulturmedium abgegeben (konditioniertes Medium). Dieses wird nach Aufkonzentration als Enzymlösung eingesetzt.

Zur Charakterisierung der enzymatischen Aktivität von endothelialer Lipase und der Wirkung von Inhibitoren wird das Phospholipase-spezifische Substrat 1,2-bis-(4,4-difluoro-5,7-dimethyl-4-bora-3a,4a-diaza-s-indacene-3-undecanoyl)-sn-glycero-3-phosphocholine, (Hersteller Molecular Probes) verwendet. Durch Hydrolyse der A1 Esterbindung dieses Phospholipids durch das Enzym wird der Fluoreszenzfarbstoff Bodipy freigesetzt, der nach Trennung durch Dünnschichtchromatographie auf einer HPTLC-Platte (Kieselgel 60, Merck) oder direkt im Reaktionsgefäß durch Messen der Fluoreszenz nachgewiesen werden kann.

Zur Herstellung der Substratlösung werden 100 µg 1,2-bis-(4,4-difluoro-5,7-dimethyl-4-bora-3a,4a-diaza-s-indacene-3-undecanoyl)-sn-glycero-3-phospho-choline (Hersteller Molecular Probes), 2,4 mg Tripalmitin (Sigma) und 7,9 mg DOP - Cholin (1,2-Dioleoyl- , sn-glycero-3-phosphocholin) in 393 µl Chloroform aufgenommen und im Anschluss 157 µl in ein frisches Reaktionsgefäß überführt. Nach Abdampfen des Lösungsmittels wird das Lipidgemisch in 4 ml 200 mM TRIS-HCl, 150 mM Natriumchlorid, pH = 7,4 durch zweimaliges Sonifizieren gelöst. Die anschließende Enzymreaktion erfolgt für 60 Minuten bei 37°C. Hierzu werden 45 µl der Substratlösung mit 1 µl inhibitor entsprechender Konzentration (gelöst in DMSO, zur Kontrolle wird reine DMSO-Lösung verwendet) und 5 µl Enzymlösung (konditioniertes Medium) inkubiert. Im Anschluß werden 3 µl des Testansatzes auf eine HPTLC-Platte (Kieselgel 60, Merck) aufgetragen und der freigesetzte Fluoreszenzfarbstoff zum Nachweis mit einem Fließmittel (Diäthylether : Petroleumbenzin : Essigsäure [78:22:1]) getrennt. Nach dem Abdampfen des Fließmittels wird die Platte in einen Fluoreszenz-Scanner eingelesen. Als Maß für die Enzymaktivität ist eine gesteigerte Freisetzung des Fluoreszenzfarbstoffes in der ungehemmten Reaktion zu beobachten.

In Abhängigkeit der verwendeten Inhibitorkonzentration ergibt sich eine Reduktion der enzymatischen Aktivität, die Inhibitiorkonzentration, bei welcher eine halbmaximale Enzymaktivität beobachtet wird, wird als IC₅₀ bezeichnet.

In diesem Test zeigten die Verbindungen der Beispiele folgende von IC₅₀ -Werte:

| Beispiel | IC₅₀ [µM] EL |
|---|---|
| 1 | 0,02 |
| 9 | 0,08 |
| 10 | 0,51 |
| 15 | 0,52 |
| 23 | 0,031 |
| 28 | 0,44 |
| 36 | 0,71 |
| 55 + 56 | 0,01* |
| 59 + 60 | 0,169* |
| 67 + 68 | 0,061* |
| 75 + 76 | 0,052* |
| 81 + 82 | 0,036* |
| 89 + 90 | 0,015* |
| 113 + 114 | 0,087* |
| 135 + 136 | 0,127* |

| | |
|---|---|
| * IC₅₀-Wert einer Mischung der angegebenen Regioisomere | |

### Verfahren zur Herstellung

Die Herstellung der erfindungsgemäßen Verbindungen der allgemeinen Formel I erfolgt nach an und für sich bekannten Methoden z.B. durch Acylierung von substituierten, bzw. unsubstituiertem Benzotriazol-derivaten II mit Carbamoylchloriden III (Methode A), oder in zwei Stufen durch Umsetzung von Benzotriazol-derivaten II mit Phosgen oder Äquivalenten wie Chlorcarbonsäuretrichlormethylester, Carbonsäureditrichlormethylester oder Chlorameisensäure-4-nitrophenylester und weiterer Umsetzung des erhaltenen Benzotriazol-carbonsäurederivats mit Aminen IV (Methode B). Ebenso können die Benzotriazol-derivate II auch mit den entsprechenden Isocyanaten V R1-N=C=O umgesetzt werden (Methode C).

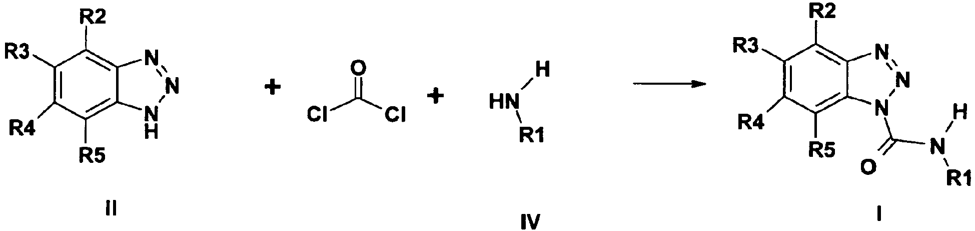

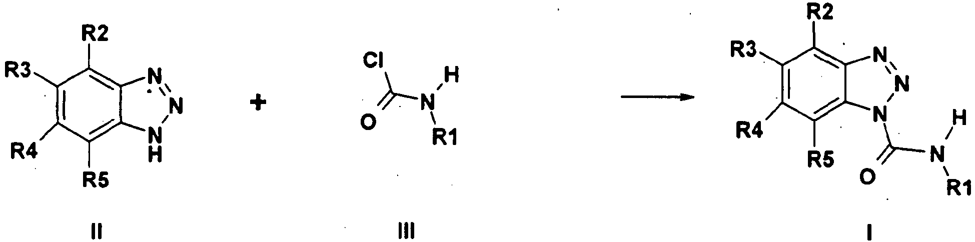

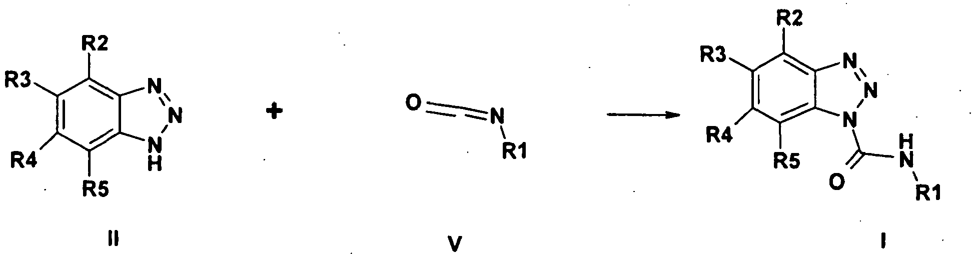

Da bei diesen Reaktionen in der Regel Säuren freigesetzt werden, empfiehlt es sich zur Beschleunigung Basen wie Pyridin, Triethylamin, Natronlauge oder Alkalicarbonate zu zusetzen. Die Reaktionen können in weiten Temperaturbereichen durchgeführt werden. In der Regel hat es sich als vorteilhaft herausgestellt, bei 0°C bis zum Siedepunkt des verwendeten Lösungsmittels zu arbeiten. Als Lösemittel kommen beispielsweise Methylenchlorid, THF, DMF, Toluol, Essigester, n-Heptan, Dioxan, Diethylether oder Pyridin zum Einsatz. Wenn unter wasserfreien Bedingungen gearbeitet wird, haben sich auch starke Basen wie Lithiumhydrid, Natriumhydrid oder Kalium-tert.-butylat in aprotischen Lösungsmitteln wie THF oder DMF bewährt.

Die als Ausgangsverbindungen II eingesetzten Benzotriazol-derivate sind kommerziell erhältlich oder lassen sich nach Literatur bekannten Verfahren herstellen (z.B. C. Flouzat, Y. Bresson, A. Mattio, J. Bonnet, G. Guillaumet J:Med.Chem 1993, 36, 497-503; F. Mutterer, C.D. Weis, J. Het. Chem. 1976, 13, 1103-1104; K.Bowden, G. Crank, W.J.Ross, J.Chem.Soc. 1968, 172-185).

Die nachfolgend aufgeführten Beispiele dienen zur Erläuterung der Erfindung, ohne diese jedoch einzuschränken.

### Beispiele

### Methode A:

Zu einer Lösung von 2 mmol 1H-Benzotriazol in Pyridin (5mL) und Dichlormethan (10 mL) wird eine Lösung des entsprechenden Carbamoylchlorids (1 mmol) in Dichlormethan (10 mL) zugegeben. Das Reaktionsgemisch wird 16 h bei RT gerührt, dann mit EtOAc (15 mL) versetzt, über Kieselgel filtriert und das Filtrat eingeengt. Das Produkt wird durch präparative HPLC gereinigt und gefriergetrocknet.

### Methode B:

### a) Herstellung einer Benzotriazol-1-carbonsäurechlorid-Lösung Zu einer Phosgenlösung (20% in Toluol; 90 mL; 182 mmol) tropft man unter

Eiskühlung eine Lösung von Benzotriazol (6 g, 50,4 mmol) und Triethylamin (7 mL) in THF (100 mL) zu. Das Eisbad wird entfernt und die Lösung noch 2 h bei RT gerührt. Das Lösungmittel wird abdestilliert und der Rückstand in Pyridin zu einem Gesamtvolumen von 25 mL aufgenommen.

### b) Umsetzung der Benzotriazolcarbonsäurechloride zu den entsprechenden Benzotriazol-1-carbonsäureamiden .

Jeweils 10 Amine (2 mmol) werden in Pyridin (5 mL) gelöst. Die Ansätze werden mit Benzotriazol-1-carbonsäurechlorid-Lösung (1 mL, ∼2 mmol) inkubiert und 16 h bei RT gerührt. Das Lösungsmittel wird abdestilliert, die Rückstände in EtOAc aufgenommen, über Kieselgel filtriert und die Filtrate im Vakuum zur Trockne eingeengt. Die Rohprodukte werden durch Flash Chromatographie oder präparative HPLC gereinigt. Das Produkt wird als Gemisch der Regioisomeren erhalten, wenn R2 ungleich R5 und R3 ungleich R4 ist, die durch bekannte Methoden, insbesondere chromatographische Methoden getrennt werden können.

Folgende Verbindungen wurden bevorzugt nach Methode B hergestellt:
(Die Identifizierung der Verbindungen erfolgte mit Massenspektrometrie und NMR-Spektroskopie).

Die mit * markierten Beispiele sind Vergleichsbeispiele.

| Beispiel | Verbindung | Mw |
|---|---|---|
| 1 | 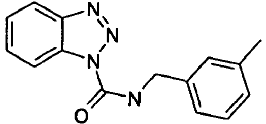 | 266,31 |
| 2 | 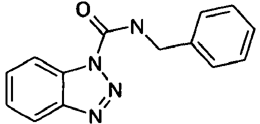 | 252,28 |
| 3* | 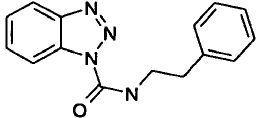 | 266,31 |
| 4 | 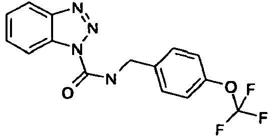 | 336,28 |
| 5* | 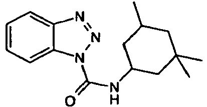 | 286,38 |
| 6* | 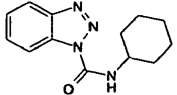 | 244,30 |
| 7 | 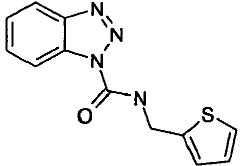 | 258,30 |
| 8 | 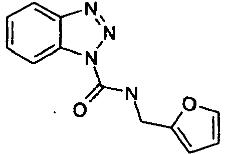 | 242,24 |
| 9 | 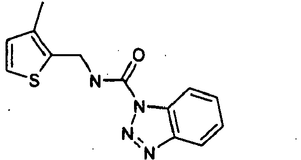 | 272,33 |
| 10 | 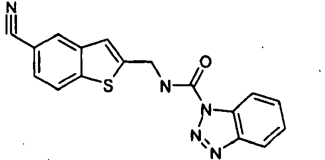 | 333,37 |
| 11 | 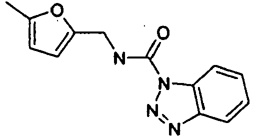 | 256,27 |
| 12 | 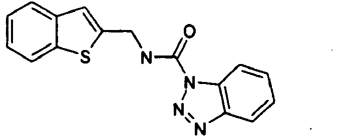 | 308,36 |
| 13 | 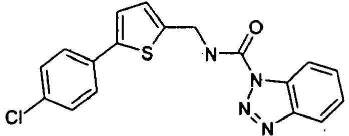 | 368,85 |
| 14 | 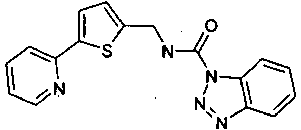 | 335,39 |
| 15 | 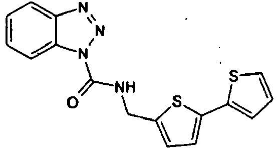 | 340,43 |
| 16 | 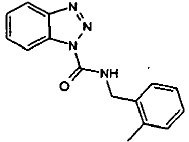 | 266,31 |
| 17 | 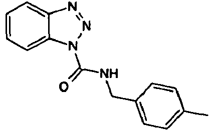 | 266,31 |
| 18* | 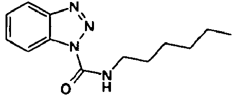 | 246,31 |
| 19 | 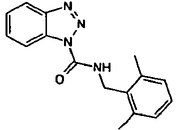 | 280,33 |
| 20* | 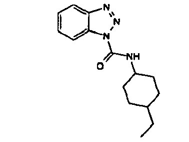 | 272,35 |
| 21* | 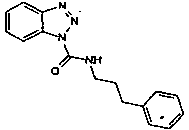 | 280,33 |
| 22 | 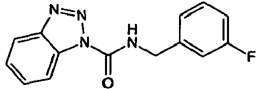 | 270,27 |
| 23 | 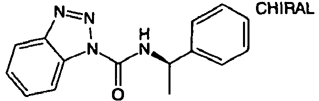 | 266,31 |
| 24 | 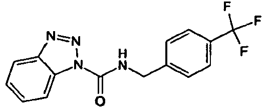 | 320,28 |
| 25 | 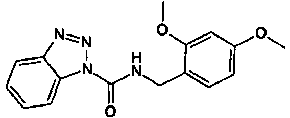 | 312,33 |
| 26 | 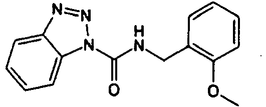 | 282,30 |
| 27 | 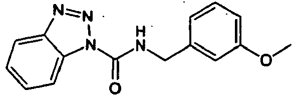 | 282,30 |
| 28 | 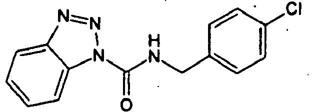 | 286,72 |
| 29 | 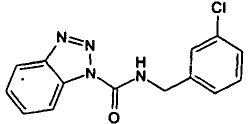 | 286,72 |
| 30 | 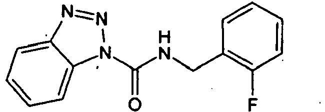 | 270,27 |
| 31 | 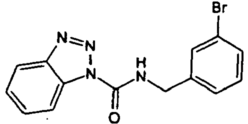 | 331,17 |
| 32 | 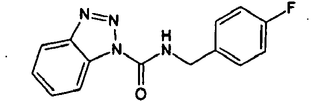 | 270,27 |
| 33 | 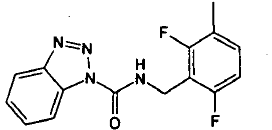 | 302,29 |
| 34 | 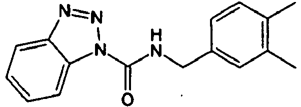 | 280,33 |
| 35 | 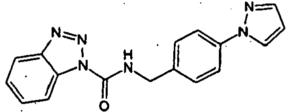 | 318,34 |
| 36 | 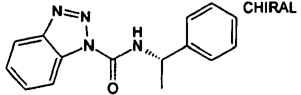 | 266,31 |
| 37 | 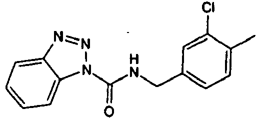 | 300,75 |
| 38 | 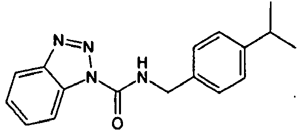 | 294,36 |
| 39 | 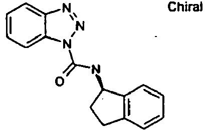 | 278,32 |
| 40 | 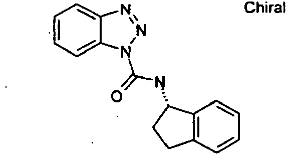 | 278,32 |
| 41 | 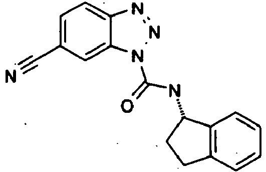 | 303,33 |
| 42 | 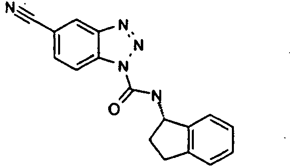 | 303,33 |
| 43 | 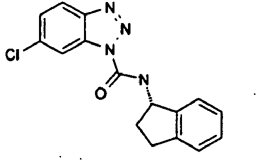 | 312,76 |
| 44 | 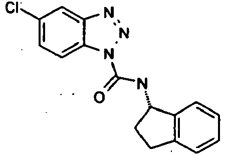 | 312,76 |
| 45 | 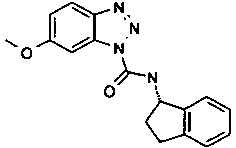 | 308,34 |
| 46 | 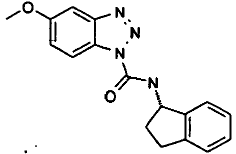 | 308,34 |
| 47 | 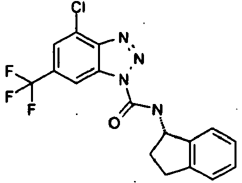 | 380,76 |
| 48 | 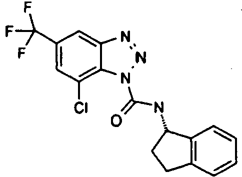 | 380,76 |
| 49 | 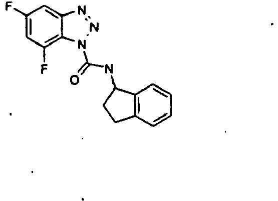 | 314,30 |
| 50 | 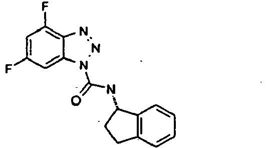 | 314,30 |
| 51 | 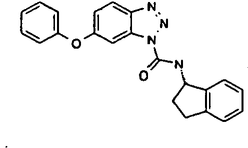 | 370,41 |
| 52 | 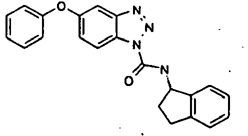 | 370,41 |
| 53 | 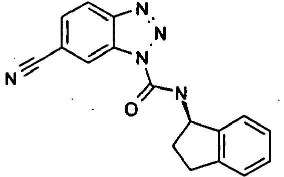 | 303,33 |
| 54 | 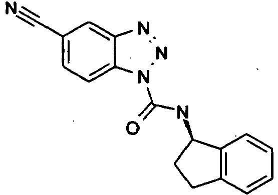 | 303,33 |
| 55 | 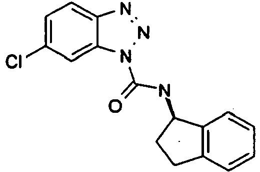 | 312,76 |
| 56 | 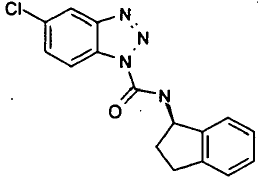 | 312,76 |
| 57 | 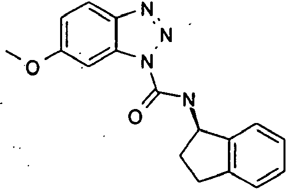 | 308,34 |
| 58 | 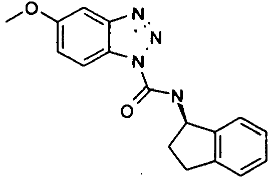 | 308,34 |
| 59 | 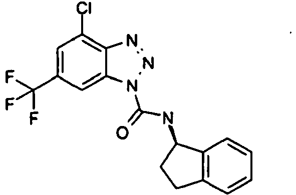 | 380,76 |
| 60 | 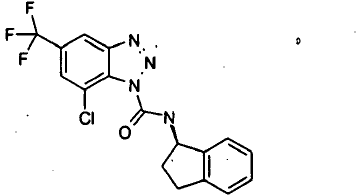 | 380,76 |
| 61 | 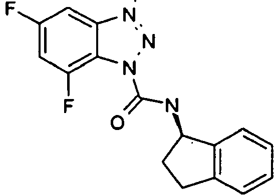 | 314,30 |
| 62 | 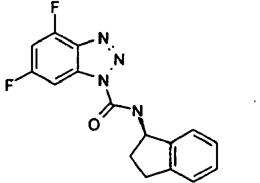 | 314,30 |
| 63 | 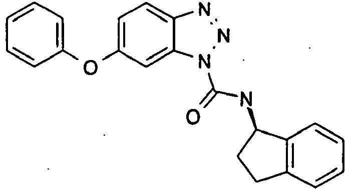 | 370,41 |
| 64 | 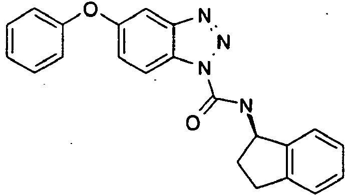 | 370,41 |
| 65 | 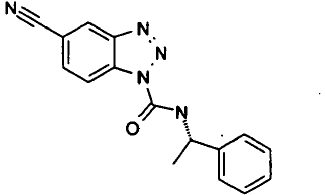 | 291,31 |
| 66 | 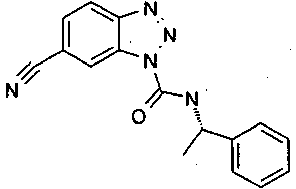 | 291,31 |
| 67 | 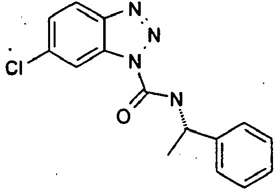 | 300,75 |
| 68 | 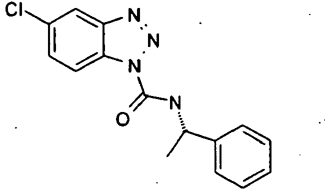 | 300,75 |
| 69 | 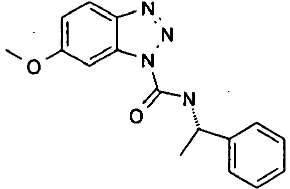 | 296,33 |
| 70 | 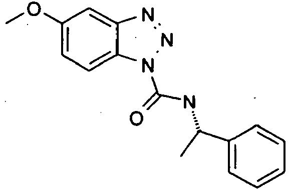 | 296,33 |
| 71 | 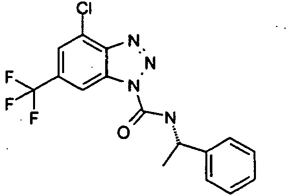 | 368,75 |
| 72 | 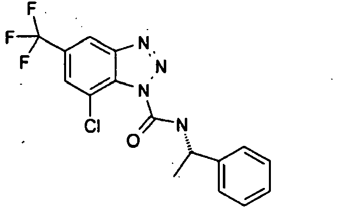 | 368,75 |
| 73 | 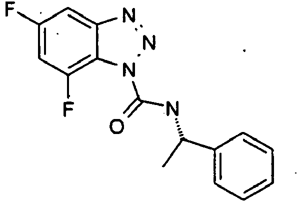 | 302,29 |
| 74 | 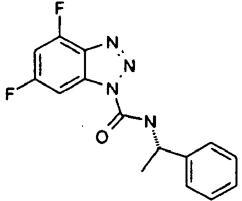 | 302,29 |
| 75 | 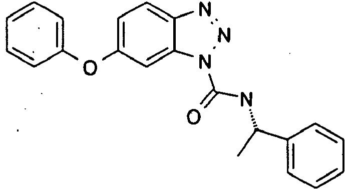 | 358,40 |
| 76 | 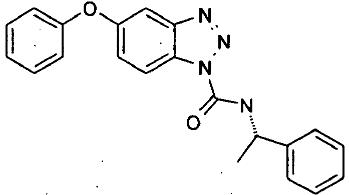 | 358,40 |
| 77 | 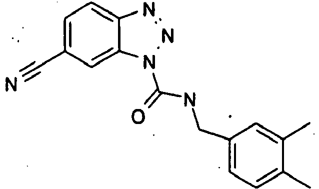 | 305,34 |
| 78 | 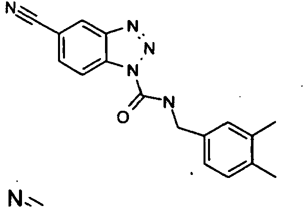 | 305,34 |
| 79 | 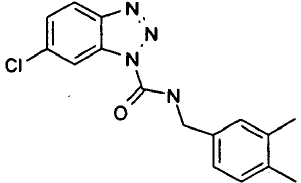 | 314,78 |
| 80 | 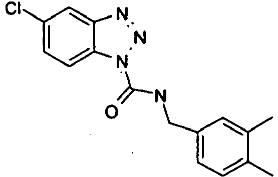 | 314,78 |
| 81 | 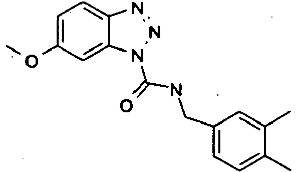 | 310,36 |
| 82 | 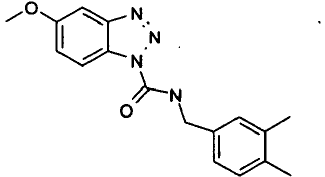 | 310,36 |
| 83 | 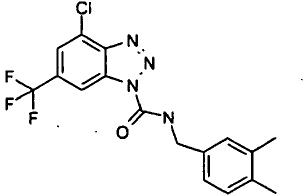 | 382,78 |
| 84 | 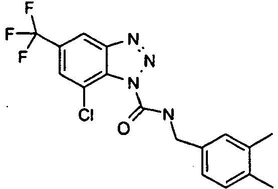 | 382,78 |
| 85 | 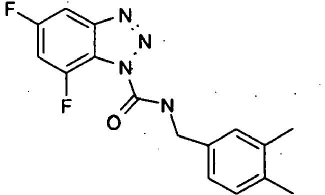 | 316,31 |
| 86 | 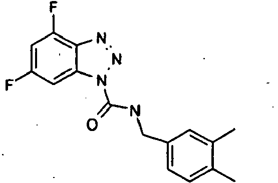 | 316,31 |
| 87 | 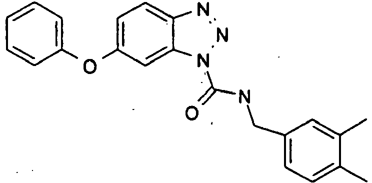 | 372,43 |
| 88 | 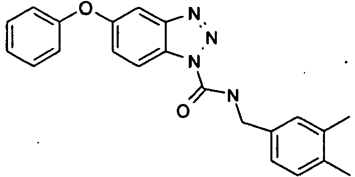 | 372,43 |
| 89 | 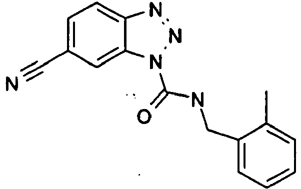 | 291,31 |
| 90 | 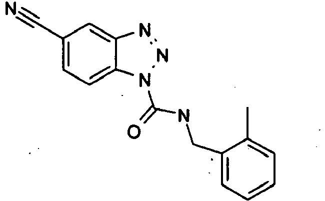 | 291,31 |
| 91 | 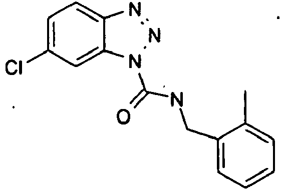 | 300,75 |
| 92 | 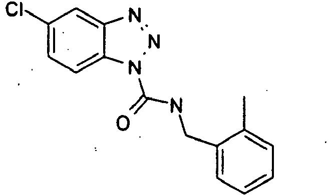 | 300,75 |
| 93 | 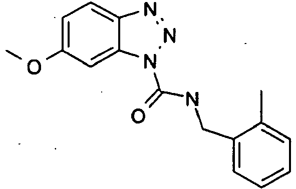 | 296,33 |
| 94 | 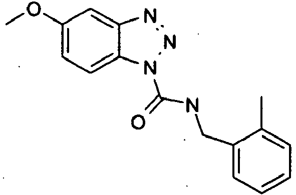 | 296,33 |
| 95 | 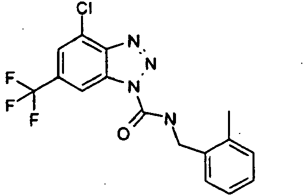 | 368,75 |
| 96 | 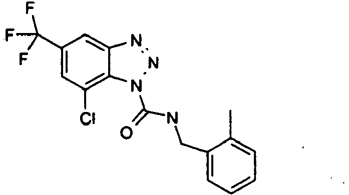 | 368,75 |
| 97 | 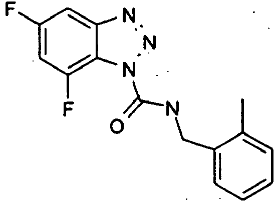 | 302,29 |
| 98 | 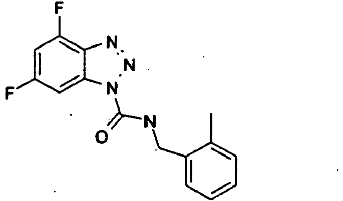 | 302,29 |
| 99 | 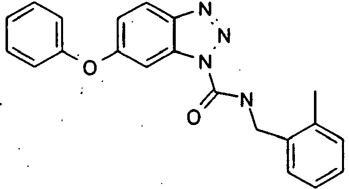 | 358,40 |
| 100 | 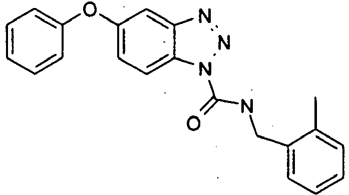 | 358,40 |
| 101 | 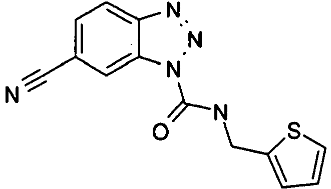 | 283,31 |
| 102 | 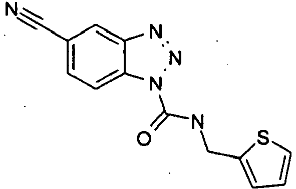 | 283,31 |
| 103 | 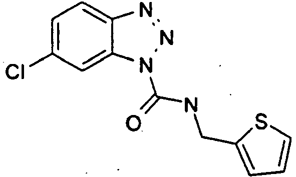 | 292,75 |
| 104 | 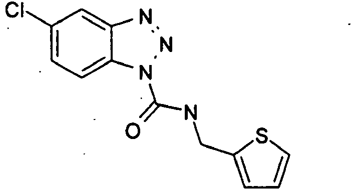 | 292,75 |
| 105 | 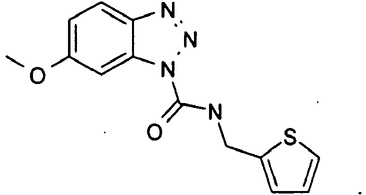 | 288,33 |
| 106 | 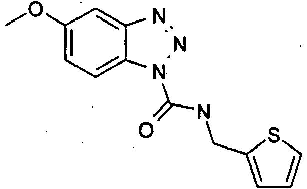 | 288,33 |
| 107 | 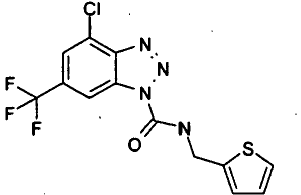 | 360,75 |
| 108 | 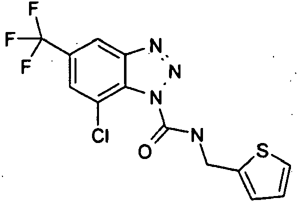 | 360,75 |
| 109 | 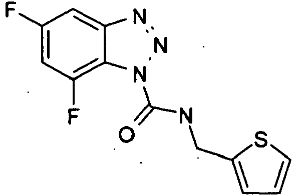 | 294,28 |
| 110 | 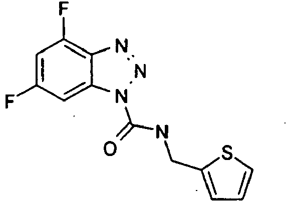 | 294,28 |
| 111 | 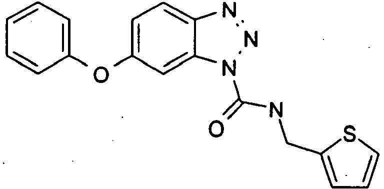 | 350,40 |
| 112 | 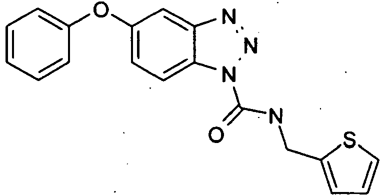 | 350,40 |
| 113 | 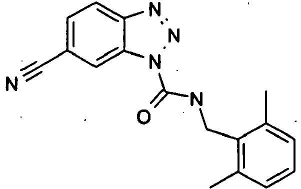 | 305,34 |
| 114 | 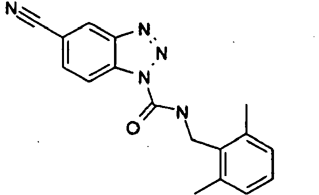 | 305,34 |
| 115 | 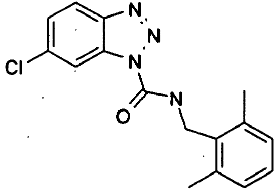 | 314,78 |
| 116 | 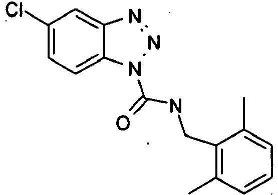 | 314,78 |
| 117 | 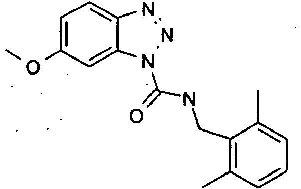 | 310,36 |
| 118 | 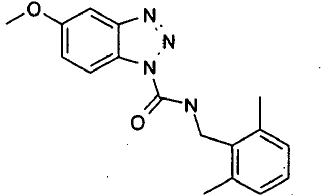 | 310,36 |
| 119 | 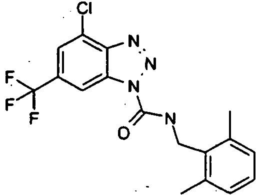 | 382,78 |
| 120 | 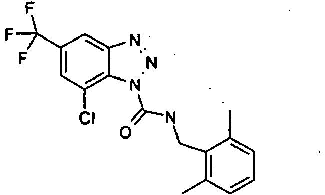 | 382,78 |
| 121 | 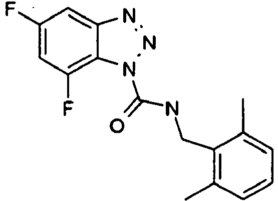 | 316,31 |
| 122 | 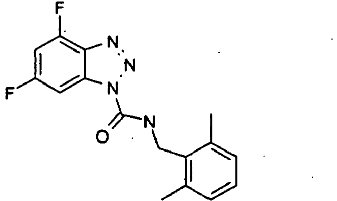 | 316,31 |
| 123 | 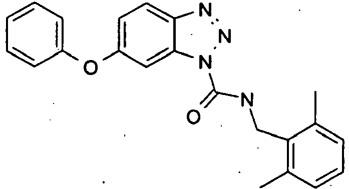 | 372,43 |
| 124 | 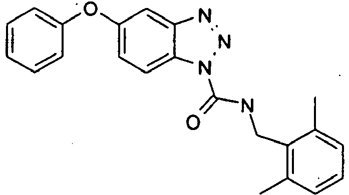 | 372,43 |
| 125 | 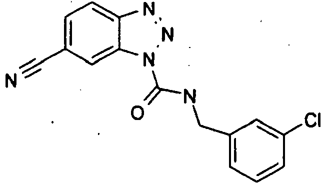 | 311,73 |
| 126 | 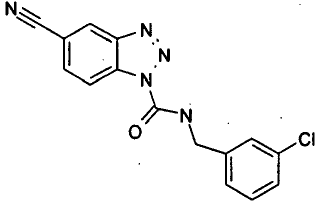 | 311,73 |
| 127 | 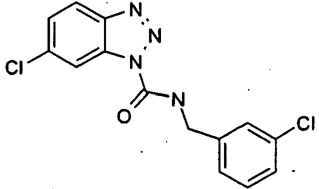 | 321,17 |
| 128 | 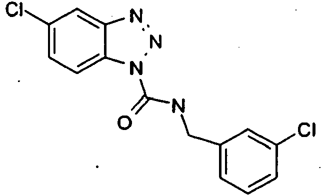 | 321,17 |
| 129 | 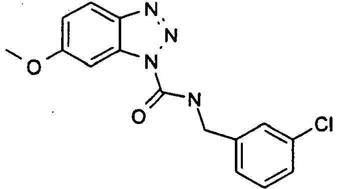 | 316,75 |
| 130 | 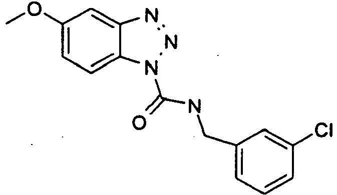 | 316,75 |
| 131 | 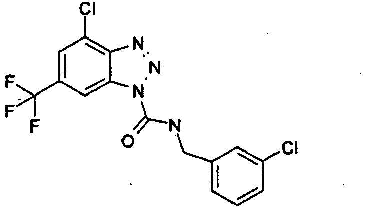 | 389,17 |
| 132 | 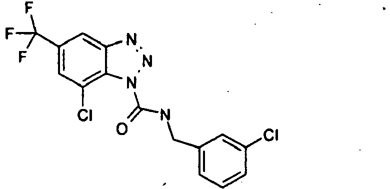 | 389,17 |
| 133 | 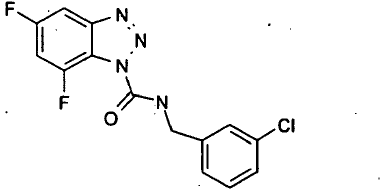 | 322,70 |
| 134 | 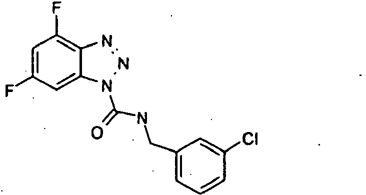 | 322,70 |
| 135 | 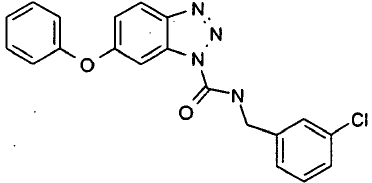 | 378,82 |
| 136 | 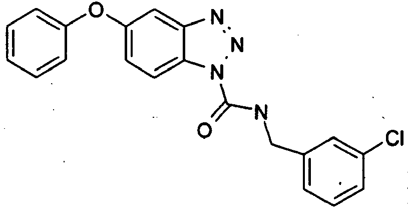 | 378,82 |

## Patentansprüche

1. Verbindung der allgemeinen Formel I 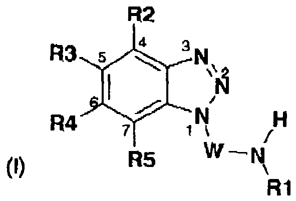 worin:
W -(C=O)-, -SO-, -SO₂-;
R1 X-Aryl, X-Heteroaryl, X-(C₅-C₁₂)-Cycloalkyl oder (C₈-C₁₄)-Bicyclus, wobei Aryl, Heteroaryl, Cycloalkyl oder Bicyclus ein oder mehrfach durch bevorzugt Halogen, (C₁-C₆)-Alkyl, (C₁-C₆)-Alkyloxy, Hydroxy, (C₁-C₆)-Alkylmercapto, Amino, (C₁-C₆)-Alkylamino, di-(C₂-C₁₂)-Alkylamino, mono-(C₁-C₆)-Alkylaminocarbonyl, di-(C₂-C₈)-Alkylaminocarbonyl, (C₁-C₆)-Alkoxycarbonyl, (C₁-C₆)-Alkylcarbonyl, Cyano, Nitro, Trifluormethyl, Trifluormethyloxy, (C₁-C₆)-Alkylsulfonyl, Aminosulfonyl substituiert und durch Y-Aryl, Y-Heteroaryl, Y-(C₃-C₁₂)-Cycloalkyl einfach substituiert sein kann, worin Aryl, Heteroaryl oder Cycloalkyl durch bevorzugt Halogen, (C₁-C₆)-Alkyl, (C₁-C₆)-Alkyloxy, Hydroxy, (C₁-C₆)-Alkylmercapto, Amino, (C₁-C₆)-Alkylamino, di-(C₂-C₁₂)-Alkylamino, mono-(C₁-C₅)-Alkylaminocarbonyl, di-(C₂-C₈)-Alkylaminocarbonyl, (C₁-C₆)-Alkoxycarbonyl, (C₁-C₆)-Alkylcarbonyl, Cyano, Nitro, Trifluormethyl, Trifluormethyloxy, (C₁-C₆)-Alkylsulfonyl, Aminosulfonyl ein bis dreifach substituiert sein kann;
X -CH₂-, welches durch Halogen, Methyl oder Hydroxy einfach substituiert sein kann;
Y eine Bindung, (C₁-C₃)-Alkylen, -O-, -NH-;
R2, R3, R4, R5 gleich oder verschieden Wasserstoff, Halogen, (C₁-C₆)-Alkyl, (C₁-C₃)-Haloalkyl, (C₁-C₃)-Alkyloxy-(C₁-C₃)-Alkylen, Hydroxy, Phenoxy, NR6R7, Cyano, Nitro, COOR6, CO-NR6R7, -S-R6, -SO-R6, -SO₂-R6, Amlnosulfonyl, Pentafluorsulfanyl, Aryl, Heteroaryl, O-Heteroaryl, (C₉-C₁₂)-Cycloalkyl, CO-R6, CO-NR6R7, O-CO-NR6R7, O-CO-(C₁-C₆)-Alkylen-CO-O-(C₁-C₆)-Alkyl, O-CO-(C₁-C₆)-Alkylen-CO-OH, O-CO-(C₁-C₆)-Alkylen-CO-NR6R7 oder unsubstituiertes oder ein oder mehrfach durch F substituiertes (C₁-C₆)-Alkyloxy;
R6, R7 gleich oder verschieden Wasserstoff, (C₁-C₆)-Alkyl, Benzyl;
bedeuten,
die tautomeren Formen der Verbindungen sowie deren physiologisch verträgliche Salze.

2. Verbindung der Formel I gemäß Anspruch 1, **dadurch**
**gekennzeichnet, dass**
W -(C=O)-
bedeutet.

3. Verbindung der Formel I gemäß Ansprüchen 1 oder 2, **dadurch**
**gekennzeichnet, dass**
W -(C=O)-;
R1 X-Aryl, X-Heteroaryl oder (C₈-C₁₄)-Bicyclus, wobei Aryl, Heteroaryl, Cycloalkyl oder Bicyclus ein oder mehrfach durch bevorzugt Halogen, (C₁-C₆)-Alkyl, (C₁-C₆)-Alkyloxy, Hydroxy, (C₁-C₆)-Alkylmercapto, Amino, (C₁-C₆)-Alkylamino, di-(C₂-C₁₂)-Alkylamino, mono-(C₁-C₆)-Alkylaminocarbonyl, di-(C₂-C₆)-Alkylaminocarbonyl, (C₁-C₆)-Alkoxycarbonyl, (C₁-C₃)-Alkylcarbonyl, Cyano, Nitro, Trifluormethyl, Trifluormethyloxy, (C₁-C₆)-Alkylsulfonyl, Aminosulfonyl substituiert und durch Y-Aryl, Y-Heteroaryl, Y-(C₃-C₁₂)-Cycloalkyl einfach substituiert sein kann, worin Aryl, Heteroaryl oder Cycloalkyl durch bevorzugt Halogen, (C₁-C₆)-Alkyl, (C₁-C₆)-Alkyloxy, Hydroxy, Amino, (C₁-C₆)-Alkylamino, di-(C₂-C₁₂)-Alkylamino, (C₁-C₆)-Alkoxycarbonyl, (C₁-C₆)-Alkylcarbonyl, Cyano, Nitro, Trifluormethyl, Trifluormethyloxy, ein bis zweifach substituiert sein kann;
X -CH₂-, welches durch Halogen, Methyl oder Hydroxy einfach substituiert sein kann;
Y eine Bindung, -O-, -NH-;
R2, R3, R4, R5 gleich oder verschieden Wasserstoff, Halogen, (C₁-C₆)-Alkyl, Trifluormethyl, Hydroxy, Amino, Cyano, Phenoxy, (C₁-C₆)-Alkylcarbonyl, (C₁-C₆)-Alkylsulfonyl, Pentafluorsulfanyl, oder unsubstituiertes oder ein oder mehrfach durch F substituiertes (C₁-C₃)-Alkyloxy;
bedeuten,
die tautomeren Formen der Verbindungen sowie deren physiologisch verträgliche Salze.

4. Verbindung der Formel I gemäß Ansprüchen 1, 2 oder 3, **dadurch**
**gekennzeichnet, dass**
W -(C=O)-;
R1 X-Phenyl, X-Heteroaryl, oder Bicyclus, wobei Aryl, Heteroaryl oder Bicyclus ein oder mehrfach durch bevorzugt Halogen, (C₁-C₆)-Alkyl, (C₁-C₆)-Alkyloxy, Hydroxy, (C₁-C₆)-Alkoxycarbonyl, (C₁-C₆)-Alkylcarbonyl, Cyano, Nitro, Trifluormethyl, Trifluormethyloxy, (C₁-C₆)-Alkylsulfonyl, Aminosulfonyl substituiert und durch Y-Phenyl, Y-Heteroaryl einfach substituiert sein kann, worin Phenyl oder Heteroaryl durch bevorzugt Halogen, (C₁-C₆)-Alkyl, (C₁-C₆)-Alkyloxy, Hydroxy, Amino, (C₁-C₆)-Alkoxycarbonyl, (C₁-C₆)-Alkylcarbonyl, Cyano, Trifluormethyl, Trifluormethyloxy, ein bis zweifach substituiert sein kann;
X -CH₂-, welches durch Fluor, Methyl oder Hydroxy substituiert sein kann;
Y eine Bindung;
R2, R3, R4, R5 gleich oder verschieden Wasserstoff, Halogen, (C₁-C₆)-Alkyl, Trifluormethyl, Hydroxy, Amino, Cyano, Phenoxy, (C₁-C₆)-Alkylcarbonyl oder unsubstituiertes oder ein oder mehrfach durch F substituiertes (C₁-C₃)-Alkyloxy;
bedeuten,
die tautomeren Formen der Verbindungen sowie deren physiologisch verträgliche Salze.

5. Verbindung der Formel I gemäß Ansprüchen 1 bis 4, **dadurch**
**gekennzeichnet, dass**
W -(C=O)-;
R1 X-Phenyl, X-Heteroaryl, oder Bicyclus der Formel Ia 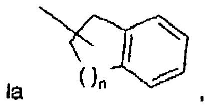 mit n =1 oder 2, wobei Phenyl, Heteroaryl oder Bicyclus der Formel Ia ein oder mehrfach durch bevorzugt Halogen, (C₁-C₈)-Alkyl, (C₁-C₆)-Alkyloxy, Hydroxy, (C₁-C₆)-Alkoxycarbonyl, (C₁-C₆)-Alkylcarbonyl, Cyano, Nitro, Trifluormethyl, Trifluormethyloxy, (C₁-C₆)-Alkylsulfonyl, Aminosulfonyl substituiert und durch Y-Phenyl, Y-Heteroaryl einfach substituiert sein kann, worin Phenyl oder Heteroaryl durch bevorzugt Halogen, (C₁-C₆)-Alkyl, (C₁-C₆)-Alkyloxy, Hydroxy, Amino, (C₁-C₆)-Alkoxycarbonyl, (C₁-C₆)-Alkylcarbonyl, Cyano, Trifluormethyl, Trifluormethyloxy ein bis zweifach substituiert sein kann;
X -CH₂-, welches durch Fluor, Methyl oder Hydroxy substituiert sein kann;
Y eine Bindung;
R2, R3, R4, R₅ gleich oder verschieden Wasserstoff, Halogen, (C₁-C₆)-Alkyl, Trifluormethyl, Hydroxy, Amino, Cyano, Phenoxy, (C₁-C₆)-Alkylcarbonyl oder unsubstituiertes oder ein oder mehrfach durch F substituiertes (C₁-C₃)-Alkyloxy;
bedeuten,
die tautomeren Formen der Verbindungen sowie deren physiologisch verträgliche Salze.

6. Verbindung der Formel I gemäß Ansprüchen 1 bis 5, **dadurch**
**gekennzeichnet, dass**
W -(C=O)-;
R1 X-Phenyl, X-Heteroaryl oder Bicyclus der Formel Ia 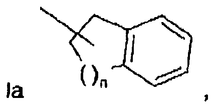 mit n =1 oder 2, wobei Phenyl, Heteroaryl oder Bicyclus der Formel la ein oder mehrfach durch bevorzugt Halogen, (C₁-C₆)-Alkyl, (C₁-C₆)-Alkyloxy, Hydroxy, (C₁-C₆)-Alkoxycarbonyl, (C₁-C₆)-Alkylcarbonyl, Cyano, Nitro, Trifluormethyl, Trifluormethyloxy, (C₁-C₆)-Alkylsulfonyl, Aminosulfonyl substituiert und durch Y-Phenyl, Y-Heteroaryl einfach substituiert sein kann, worin Phenyl oder Heteroaryl durch bevorzugt Halogen, (C₁-C₆)-Alkyl, (C₁-C₆)-Alkyloxy, Hydroxy, Amino, (C₁-C₆)-Alkoxycarbonyl, (C₁-C₆)-Alkylcarbonyl, Cyano, Trifluormethyl, Trifluormethyloxy ein bis zweifach substituiert sein kann;
X -CH₂-, welches durch Methyl substituiert sein kann;
Y eine Bindung;
R2, R3, R4, R5 gleich oder verschieden Wasserstoff, Halogen, (C₁-C₆)-Alkyl, Trifluormethyl, Hydroxy, Amino, Cyano, Phenoxy, (C₁-C₆)-Alkylcarbonyl oder unsubstituiertes oder ein oder mehrfach durch F substituiertes (C₁-C₃)-Alkyloxy;
bedeuten.

7. Verbindung der Formel I gemäß Ansprüchen 1 bis 6, **dadurch**
**gekennzeichnet, dass**
W -(C=O)-;
R1 X-Phenyl, X-Thienyl, X-Furan, X-Benzothienyl, Indanyl oder Tetrahydronaphtyl, wobei Phenyl, Thienyl, Furan, Benzothienyl, Indanyl oder Tetrahydronaphtyl ein-, zwei- oder dreifach durch F, Cl, Br, Methyl, Ethyl, Isopropyl, Methoxy, Ethoxy, Hydroxy, CO-OCH₃, CO-CH₃, Cyano, Nitro, Trifluormethyl, Trifluormethyloxy substituiert und
durch Y-Phenyl, Y-Thienyl, Y-Pyridyl, Y-Pyrazolyl einfach substituiert sein kann, worin Phenyl, Thienyl, Pyridyl oder Pyrazolyl durch bevorzugt F, Cl, Br, Methyl, Methoxy, Hydroxy, Amino, CO-OCH₃, CO-CH₃, Cyano, Trifluormethyl, Trifluormethyloxy ein bis zweifach substituiert sein kann;
X -CH₂-, welches durch Methyl substituiert sein kann;
Y eine Bindung;
R2, R3, R4, R5 gleich oder verschieden Wasserstoff, F, Cl, Methoxy, Trifluormethyl, Cyano, Phenoxy;
bedeuten.

8. Verbindung der Formel I gemäß Ansprüchen 1 bis 7, **dadurch**
**gekennzeichnet, dass**
W -(C=O)-,
R1 X-Phenyl, X-Thienyl, X-Furan, X-Benzothienyl oder Indanyl, wobei Phenyl, Thienyl, X-Furan, X-Benzothienyl ein oder zweifach durch F, Cl, Br, Methyl, Isopropyl, Methoxy, Cyano, Trifluormethyl substituiert und durch Y-Phenyl, Y-Thienyl, Y-Pyridyl, Y-Pyrazolyl einfach substituiert sein kann, worin Phenyl durch Cl substituiert sein kann;
X -CH₂-, welches durch Methyl substituiert sein kann;
Y eine Bindung;
R2 Wasserstoff, F, Cl;
R3 Wasserstoff, F, Cl, Methoxy, Trifluormethyl, Cyano, Phenoxy;
R4 Wasserstoff, F, Cl, Methoxy, Trifluormethyl, Cyano, Phenoxy;
R5 Wasserstoff, F, Cl;
bedeuten.

9. Verbindung der Formel I gemäß Ansprüchen 1 bis 8, **dadurch**
**gekennzeichnet, dass**
R2, R3, R4, R5 Wasserstoff;
bedeuten.

10. Verbindung der Formel I gemäß Ansprüchen 1 bis 8, **dadurch gekennzeichnet, dass**
R2, R3, R5 Wasserstoff;
und
R4 nicht Wasserstoff; oder
R2, R4, R5 Wasserstoff;
und
R3 nicht Wasserstoff;
bedeuten.

11. Arzneimittel enthaltend eine oder mehrere Verbindungen der Formel I gemäß den Ansprüchen 1 bis 10.

12. Verwendung der Verbindungen der Formel I gemäß den Ansprüchen 1 bis 10 zur Herstellung eines Medikaments zur Behandlung und/oder Prävention von Störungen des Fettsäurestoffwechsels und Glucoseverwertungsstörungen.

13. Verwendung der Verbindungen der Formel I gemäß den Ansprüchen 1 bis 10 zur Herstellung eines Medikaments zur Behandlung und/oder Prävention von Störungen, bei denen Insulin Resistenz eine Rolle spielt.

14. Verwendung der Verbindungen der Formel I gemäß den Ansprüchen 1 bis 10 zur Herstellung eines Medikaments zur Behandlung und/oder Prävention von Diabetes mellitus und der damit verbundenen Folgeerkrankungen.

15. Verwendung der Verbindungen der Formel I gemäß den Ansprüchen 1 bis 10 zur Herstellung eines Medikaments zur Behandlung und/oder Prävention von Dyslipidämlen und deren Folgen.

16. Verwendung der Verbindungen der Formel I gemäß den Ansprüchen 1 bis 10 zur Herstellung eines Medikaments zur Behandlung und/oder Prävention von Zuständen, die mit dem Metabolischen Syndrom assoziert sind.

17. Verwendung der Verbindungen der Formel I gemäB den Ansprüchen 1 bis10 zur Herstellung eines Medikaments zur Behandlung und/oder Prävention von Zuständen, die mit erniedrigtem HDL-Spiegel verbunden sind.

18. Verwendung der Verbindungen der Formel I gemäß den Ansprüchen 1 bis 10 zur Herstellung eines Medikaments zur Behandlung und/oder Prävention von atherosklerotlschen Erkrankungen.

19. Verwendung der Verbindungen der Formel I gemäß den Ansprüchen 1 bis 10 in Kombination mit mindestens einem weiteren Wirkstoff zur Herstellung eines Medikaments zur Behandlung und/oder Prävention von Störungen, bei denen Insulin Resistenz eine Rolle spielt.

20. Verfahren zur Herstellung eines Arzneimittels enthaltend eine oder mehrere der Verbindungen der Formel I gemäß den Ansprüchen 1 bis 10, **dadurch gekennzeichnet, dass** diese mit einem pharmazeutisch geeigneten Träger vermischt wird und diese Mischung in eine für die Verabreichung geeignete Form gebracht wird.

21. Verfahren zur Herstellung von Verbindungen der allgemeinen Formel I gemäß den Ansprüchen 1 bis 10, **dadurch gekennzeichnet, dass** Benzotriazol-derivate der Formel II
a) mit Carbamoylchloriden der Formel III acyliert werden;
oder
b) in zwei Stufen zunächst mit Phosgen oder Äquivalenten wie Chlorcarbonsäuretrichlormethylester, Carbonsäureditrichlormethylester oder Chlorameisensäure-4-nitrophenylester und einem zweiten Schritt mit Aminen der Formel IV umgesetzt werden,
oder
c) mit Isocyanaten der Formel V: O=C=N-R1 umgesetzt werden, worin die Substituenten die oben genannten Bedeutungen haben, und gegebenenfalls die entstandenen Regioisomere mit bekannten Methoden, insbesondere chromatographischen Methoden getrennt werden.

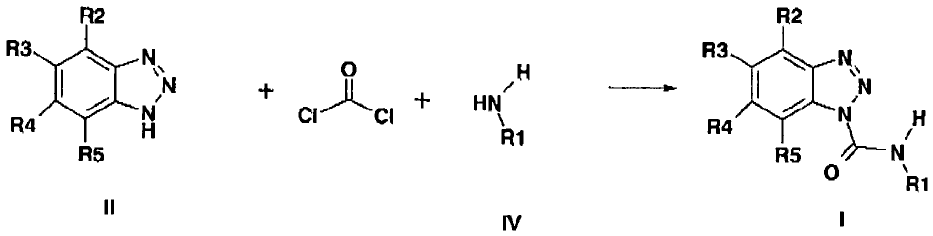

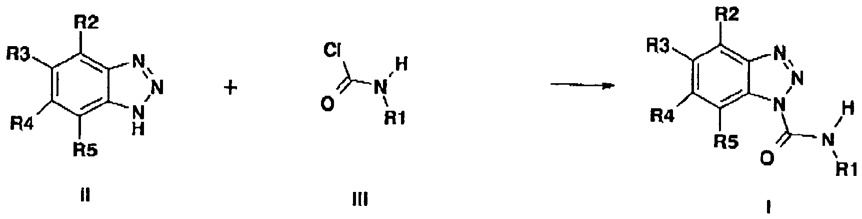

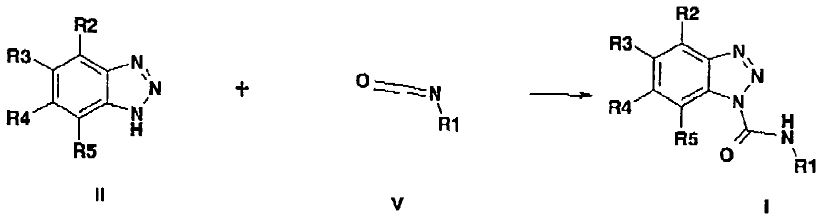

## Claims

1. A compound of the formula I 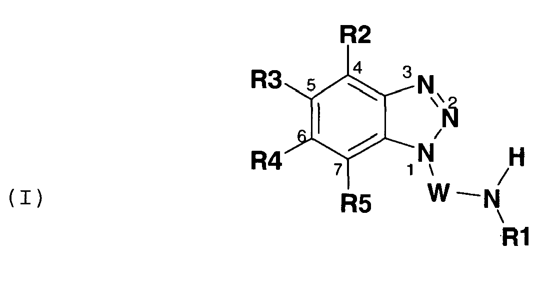 in which
W is -(C=O)-, -SO-, -SO₂-;
R1 is X-aryl, X-heteroaryl, X-(C₅-C₁₂)-cycloalkyl or (C₈-C₁₄)-bicycle, where aryl, heteroaryl, cycloalkyl or bicycle may be substituted one or more times by preferably halogen, (C₁-C₆)-alkyl, (C₁-C₆)-alkyloxy, hydroxy, (C₁-C₆)-alkylmercapto, amino, (C₁-C₆)-alkylamino, di-(C₂-C₁₂)-alkylamino, mono-(C₁-C₆)-alkylaminocarbonyl, di-(C₂-C₈)-alkylaminocarbonyl, (C₁-C₆)-alkoxycarbonyl, (C₁-C₆)-alkylcarbonyl, cyano, nitro, trifluoromethyl, trifluoromethyloxy, (C₁-C₆)-alkylsulfonyl, aminosulfonyl, and may be substituted once by Y-aryl, Y-heteroaryl, Y-(C₃-C₁₂)-cycloalkyl, in which aryl, heteroaryl or cycloalkyl may be substituted one to three times by preferably halogen, (C₁-C₆)-alkyl, (C₁-C₆)-alkyloxy, hydroxy, (C₁-C₆)-alkylmercapto, amino, (C₁-C₆)-alkylamino, di- (C₂-C₁₂) -alkylamino, mono- (C₁-C₆)-alkylaminocarbonyl, di-(C₂-C₈)-alkylaminocarbonyl, (C₁-C₆)-alkoxycarbonyl, (C₁-C₆)-alkylcarbonyl, cyano, nitro, trifluoromethyl, trifluoromethyloxy, (C₁-C₆)-alkylsulfonyl, aminosulfonyl;
X is -CH₂-, which may be substituted once by halogen, methyl or hydroxy;
Y is a bond, (C₁-C₃)-alkylene, -0-, -NH-;
R2, R3, R4, R5 are identically or differently hydrogen, halogen, (C₁-C₆)-alkyl, (C₁-C₃)-haloalkyl, (C₁-C₃)-alkyloxy-(C₁-C₃)-alkylene, hydroxy, phenoxy, NR6R7, cyano, nitro, COOR6, CO-NR6R7, -S-R6, -SO-R6, -SO₂-R6, aminosulfonyl, pentafluorosulfanyl, aryl, heteroaryl, O-heteroaryl, (C₃-C₁₂)-cycloalkyl, CO-R6, CO-NR6R7, O-CO-NR6R7, O-CO-(C₁-C₆)-alkylene-CO-O-(C₁-C₆)-alkyl, O-CO-(C₁-C₆)-alkylene-CO-OH, O-CO-(C₁-C₆)-alkylene-CO-NR6R7 or unsubstituted or mono- or poly-F-substituted (C₁-C₆)-alkyloxy;
R6, R7 are identically or differently hydrogen, (C₁-C₆)-alkyl, benzyl;
the tautomeric forms of the compounds and their physiologically tolerated salts.

2. A compound of the formula I as claimed in claim 1, wherein
W is -(C=O)-.

3. A compound of the formula I as claimed in claims 1 or 2, wherein
W is -(C=O)-;
R1 is X-aryl, X-heteroaryl or (C₈-C₁₄)-bicycle, where aryl, heteroaryl, cycloalkyl or bicycle may be substituted one or more times by preferably halogen, (C₁-C₆)-alkyl, (C₁-C₆)-alkyloxy, hydroxy, (C₁-C₆)-alkylmercapto, amino, (C₁-C₆)-alkylamino, di-(C₂-C₁₂)-alkylamino, mono-(C₁-C₆)-alkylaminocarbonyl, di-(C₂-C₈)-alkylaminocarbonyl, (C₁-C₆)-alkoxycarbonyl, (C₁-C₆)-alkylcarbonyl, cyano, nitro, trifluoromethyl, trifluoromethyloxy, (C₁-C₆)-alkylsulfonyl, aminosulfonyl, and may be substituted once by Y-aryl, Y-heteroaryl, Y-(C₃-C₁₂)-cycloalkyl, in which aryl, heteroaryl or cycloalkyl may be substituted once to twice by preferably halogen, (C₁-C₆)-alkyl, (C₁-C₆)-alkyloxy, hydroxy, amino, (C₁-C₆)-alkylamino, di-(C₂-C₁₂)-alkylamino, (C₁-C₆)-alkoxycarbonyl, (C₁-C₆)-alkylcarbonyl, cyano, nitro, trifluoromethyl, trifluoromethyloxy;
X is -CH₂-, which may be substituted once by halogen, methyl or hydroxy;
Y is a bond, -O-, -NH-;
R2, R3, R4, R5 are identically or differently hydrogen, halogen, (C₁-C₆)-alkyl, trifluoromethyl, hydroxy, amino, cyano, phenoxy, (C₁-C₆)-alkylcarbonyl, (C₁-C₆)-alkylsulfonyl, pentafluorosulfanyl, or unsubstituted or mono- or poly-F-substituted (C₁-C₃)-alkyloxy;
the tautomeric forms of the compounds and their physiologically tolerated salts.

4. A compound of the formula I as claimed in claims 1, 2 or 3, wherein
W is -(C=O)-;
R1 is X-phenyl, X-heteroaryl, or bicycle, where aryl, heteroaryl or bicycle may be substituted one or more times by preferably halogen, (C₁-C₆)-alkyl, (C₁-C₆)-alkyloxy, hydroxy, (C₁-C₆)-alkoxycarbonyl, (C₁-C₆)-alkylcarbonyl, cyano, nitro, trifluoromethyl,
trifluoromethyloxy, (C₁-C₆)-alkylsulfonyl, aminosulfonyl, and may be substituted once by Y-phenyl, Y-heteroaryl, in which phenyl or heteroaryl may be substituted once to twice by preferably halogen, (C₁-C₆)-alkyl, (C₁-C₆)-alkyloxy, hydroxy, amino, (C₁-C₆)-alkoxycarbonyl, (C₁-C₆)-alkylcarbonyl, cyano, trifluoromethyl, trifluoromethyloxy;
X is -CH₂-, which may be substituted by fluorine, methyl or hydroxy;
Y is a bond;
R2, R3, R4, R5 are identically or differently hydrogen, halogen, (C₁-C₆)-alkyl, trifluoromethyl, hydroxy, amino, cyano, phenoxy, (C₁-C₆)-alkylcarbonyl or unsubstituted or mono- or poly-F-substituted (C₁-C₃)-alkyloxy;
the tautomeric forms of the compounds and their physiologically tolerated salts.

5. A compound of the formula I as claimed in claims 1 to 4, wherein
W is -(C=O)-;
R1 is X-phenyl, X-heteroaryl, or bicycle of the formula Ia 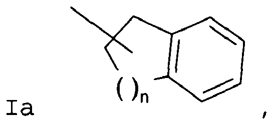 with n =1 or 2, where phenyl, heteroaryl or bicycle of the formula Ia may be substituted one or more times by preferably halogen, (C₁-C₆)-alkyl, (C₁-C₆)-alkyloxy, hydroxy, (C₁-C₆)-alkoxycarbonyl, (C₁-C₆)-alkylcarbonyl, cyano, nitro, trifluoromethyl, trifluoromethyloxy, (C₁-C₆)-alkylsulfonyl, aminosulfonyl, and may be substituted once by Y-phenyl, Y-heteroaryl, in which phenyl or heteroaryl may be substituted once to twice by preferably halogen, (C₁-C₆)-alkyl, (C₁-C₆)-alkyloxy, hydroxy, amino, (C₁-C₆)-alkoxycarbonyl, (C₁-C₆)-alkylcarbonyl, cyano, trifluoromethyl, trifluoromethyloxy;
X is -CH₂-, which may be substituted by fluorine, methyl or hydroxy;
Y is a bond;
R2, R3, R4, R5 are identically or differently hydrogen, halogen, (C₁-C₆)-alkyl, trifluoromethyl, hydroxy, amino, cyano, phenoxy, (C₁-C₆)-alkylcarbonyl or unsubstituted or mono- or poly-F-substituted (C₁-C₃)-alkyloxy;
the tautomeric forms of the compounds and their physiologically tolerated salts.

6. A compound of the formula I as claimed in claims 1 to 5, wherein
W is -(C=O)-;
R1 is X-phenyl, X-heteroaryl, or bicycle of the formula Ia 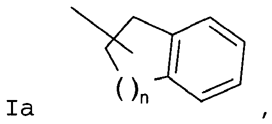 with n =1 or 2, where phenyl, heteroaryl or bicycle of the formula Ia may be substituted one or more times by preferably halogen, (C₁-C₆)-alkyl, (C₁-C₆)-alkyloxy, hydroxy, (C₁-C₆)-alkoxycarbonyl, (C₁-C₆)-alkylcarbonyl, cyano, nitro, trifluoromethyl, trifluoromethyloxy, (C₁-C₆)-alkylsulfonyl, aminosulfonyl, and may be substituted once by Y-phenyl, Y-heteroaryl, in which phenyl or heteroaryl may be substituted once to twice by preferably halogen, (C₁-C₆)-alkyl, (C₁-C₆)-alkyloxy, hydroxy, amino, (C₁-C₆)-alkoxycarbonyl, (C₁-C₆)-alkylcarbonyl, cyano, trifluoromethyl, trifluoromethyloxy;
X is -CH₂- which may be substituted by methyl;
Y is a bond;
R2, R3, R4, R5 are identically or differently hydrogen, halogen, (C₁-C₆)-alkyl, trifluoromethyl, hydroxy, amino, cyano, phenoxy, (C₁-C₆)-alkylcarbonyl or unsubstituted or mono- or poly-F-substituted (C₁-C₃)-alkyloxy.

7. A compound of the formula I as claimed in claims 1 to 6, wherein
W is -(C=O)-;
R1 is X-phenyl, X-thienyl, X-furan, X-benzothienyl, indanyl or tetrahydronaphthyl, where phenyl, thienyl, furan, benzothienyl, indanyl or tetrahydronaphthyl may be substituted once, twice or three times by F, Cl, Br, methyl, ethyl, isopropyl, methoxy, ethoxy, hydroxy, CO-OCH₃, CO-CH₃, cyano, nitro, trifluoromethyl, trifluoromethyloxy and may be substituted once by Y-phenyl, Y-thienyl, Y-pyridyl, Y-pyrazolyl, in which phenyl, thienyl, pyridyl or pyrazolyl may be substituted once to twice by preferably F, Cl, Br, methyl, methoxy, hydroxy, amino, CO-OCH₃, CO-CH₃, cyano, trifluoromethyl, trifluoromethyloxy;
X is -CH₂- which may be substituted by methyl;
Y is a bond;
R2, R3, R4, R5 are identically or differently hydrogen, F, Cl, methoxy, trifluoromethyl, cyano, phenoxy.

8. A compound of the formula I as claimed in claims 1 to 7, wherein
W is -(C=O)-;
R1 is X-phenyl, X-thienyl, X-furan, X-benzothienyl or indanyl, where phenyl, thienyl, X-furan, X-benzothienyl may be substituted once or twice by F, Cl, Br, methyl, isopropyl, methoxy, cyano, trifluoromethyl and may be substituted once by Y-phenyl, Y-thienyl, Y-pyridyl, Y-pyrazolyl, in which phenyl may be substituted by Cl;
X is -CH₂-, which may be substituted by methyl;
Y is a bond;
R2 is hydrogen, F, Cl;
R3 is hydrogen, F, Cl, methoxy, trifluoromethyl, cyano, phenoxy;
R4 is hydrogen, F, Cl, methoxy, trifluoromethyl, cyano, phenoxy;
R5 is hydrogen, F, Cl.

9. A compound of the formula I as claimed in claims 1 to 8, wherein
R2, R3, R4, R5 are hydrogen.

10. A compound of the formula I as claimed in claims 1 to 8, wherein
R2, R3, R5 are hydrogen;
and
R4 is not hydrogen; or
R2, R4, R5 are hydrogen;
and
R3 is not hydrogen.

11. A medicament comprising one or more compounds of the formula I as claimed in claims 1 to 10.

12. The use of the compounds of the formula I as claimed in claims 1 to 10 for producing a medicament for the treatment and/or prevention of disorders of fatty acid metabolism and glucose utilization disorders.

13. The use of the compounds of the formula I as claimed in claims 1 to 10 for producing a medicament for the treatment and/or prevention of disorders in which insulin resistance is involved.

14. The use of the compounds of the formula I as claimed in claims 1 to 10 for producing a medicament for the treatment and/or prevention of diabetes mellitus and the sequelae associated therewith.

15. The use of the compounds of the formula I as claimed in claims 1 to 10 for producing a medicament for the treatment and/or prevention of dyslipidemias and their sequelae.

16. The use of the compounds of the formula I as claimed in claims 1 to 10 for producing a medicament for the treatment and/or prevention of conditions associated with the metabolic syndrome.

17. The use of the compounds of the formula I as claimed in claims 1 to 10 for producing a medicament for the treatment and/or prevention of conditions associated with reduced HDL level.

18. The use of the compounds of the formula I as claimed in claims 1 to 10 for producing a medicament for the treatment and/or prevention of atherosclerotic disorders.

19. The use of the compounds of the formula I as claimed in claims 1 to 10 in combination with at least one further active ingredient for producing a medicament for the treatment and/or prevention of disorders in which insulin resistance is involved.

20. A process for producing a medicament comprising one or more of the compounds of the formula I as claimed in claims 1 to 10, which comprises mixing the latter with a pharmaceutically suitable carrier, and converting this mixture into a form suitable for administration.

21. A process for preparing compounds of the formula I as claimed in claims 1 to 10, which comprises benzotriazole derivatives of the formula II
a) being acylated with carbamoyl chlorides of the formula III;
or
b) in two stages being reacted first with phosgene or equivalents such as trichloromethyl chlorocarbonate, ditrichloromethyl carbonate or 4-nitrophenyl chloroformate and in a second step with amines of the formula IV,
or
c) being reacted with isocyanates of the formula V: O=C=N-R1,
in which the substituents have the abovementioned meanings, and where appropriate separating the resulting regioisomers by known methods, in particular chromatographic methods

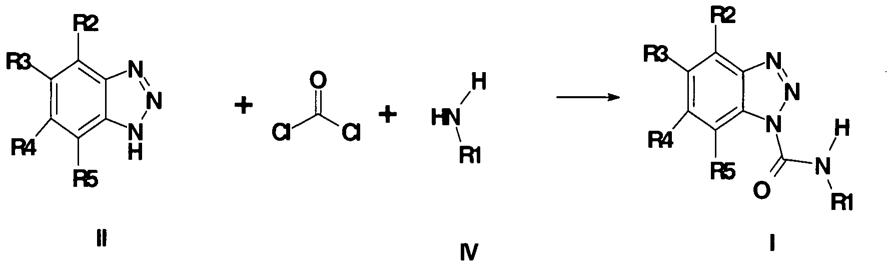

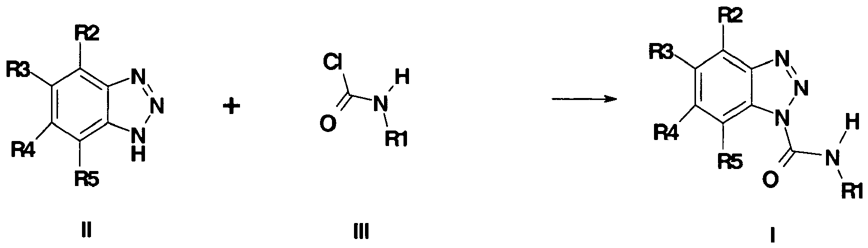

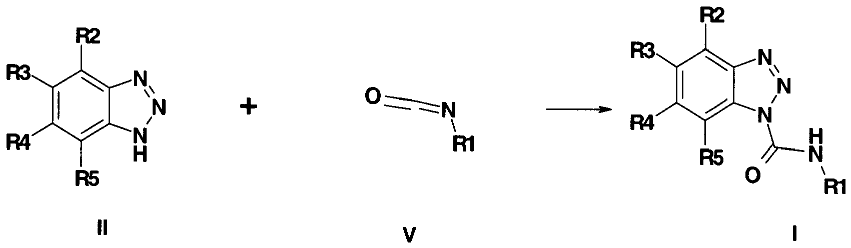

## Revendications

1. Composé de formule générale I 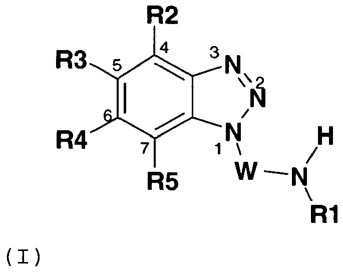 dans laquelle
W est -(C=O)-, -SO-, -SO₂- ;
R1 est X-aryle, X-hétéroaryle, X-(cycloalkyle en C₅-C₁₂) ou un bicycle en C₈-C₁₄, où l'aryle, l'hétéroaryle, le cycloalkyle ou le bicycle peuvent être substitués une ou plusieurs fois par, de préférence, un halogène, un alkyle en C₁-C₆, un alkyloxy en C₁-C₆, un hydroxy, un (alkyle en C₁-C₆)mercapto, un amino, un (alkyle en C₁-C₆)amino, un di(alkyle en C₂-C₁₂)amino, un mono(alkyle en C₁-C₆)aminocarbonyle, un di(alkyle en C₂-C₈)aminocarbonyle, un (alcoxy en C₁-C₆)carbonyle, un (alkyle en C₁-C₆)carbonyle, un cyano, un nitro, un trifluorométhyle, un trifluorométhyloxy, un (alkyle en C₁-C₆)sulfonyle, un aminosulfonyle, et peuvent être substitués une fois par Y-aryle, Y-hétéroaryle, Y-(cycloalkyle en C₃-C₁₂), où l'aryle, l'hétéroaryle ou le cycloalkyle peuvent être substitués une à trois fois par, de préférence, un halogène, un alkyle en C₁-C₆, un alkyloxy en C₁-C₆, un hydroxy, un (alkyle en C₁-C₆)mercapto, un amino, un (alkyle en C₁-C₆)amino, un di (alkyle en C₂-C₁₂)amino, un mono (alkyle en C₁-C₆) aminocarbonyle, un di(alkyle en C₂-C₈)aminocarbonyle, un (alcoxy en C₁-C₆)carbonyle, un (alkyle en C₁-C₆)carbonyle, un cyano, un nitro, un trifluorométhyle, un trifluorométhyloxy, un (alkyle en C₁-C₆)sulfonyle, un aminosulfonyle ;
X est -CH₂-, qui peut être substitué une fois par un halogène, un méthyle ou un hydroxy ;
Y est une liaison, un alkylène en C₁-C₃, -O-, -NH- ;
R2, R3, R4, R5 sont, identiques ou différents, un hydrogène, un halogène, un alkyle en C₁-C₆, un halogénoalkyle en C₁-C₃, un (alkyloxy en C₁-C₃)-(alkylène en C₁-C₃), un hydroxy, un phénoxy, NR6R7, un cyano, un nitro, COOR6, CO-NR6R7, -S-R6, -SO-R6, -SO₂-R6, un aminosulfonyle, un pentafluorosulfanyle, un aryle, un hétéroaryle, O-hétéroaryle, un cycloalkyle en C₃-C₁₂, CO-R6, CO-NR6R7, O-CO-NR6R7, O-CO-(alkylène en C₁-C₆)-CO-O-(alkyle en C₁-C₆), O-CO-(alkylène en C₁-C₆)-CO-OH, O-CO-(alkylène en C₁-C₆)-CO-NR6R7 ou un alkyloxy en C₁-C₆ non substitué ou substitué une ou plusieurs fois par F ;
R6, R7 sont, identiques ou différents, un hydrogène, un alkyle en C₁-C₆, un benzyle ;
les formes tautomères des composés et leurs sels physiologiquement tolérés.

2. Composé de formule I selon la revendication 1, **caractérisé en ce que**
W est -(C=O)-.

3. Composé de formule I selon les revendications 1 ou 2, **caractérisé en ce que**
W est -(C=O)- ;
R1 est X-aryle, X-hétéroaryle ou un bicycle en C₈-C₁₄, où l'aryle, l'hétéroaryle, le cycloalkyle ou le bicycle peuvent être substitués une ou plusieurs fois par, de préférence, un halogène, un alkyle en C₁-C₆, un alkyloxy en C₁-C₆, un hydroxy, un (alkyle en C₁-C₆)mercapto, un amino, un (alkyle en C₁-C₆)amino, un di (alkyle en C₂-C₁₂)amino, un mono(alkyle en C₁-C₆)aminocarbonyle, un di(alkyle en C₂-C₈)aminocarbonyle, un (alcoxy en C₁-C₆)carbonyle, un (alkyle en C₁-C₆) carbonyle, un cyano, un nitro, un trifluorométhyle, un trifluorométhyloxy, un (alkyle en C₁-C₆)sulfonyle, un aminosulfonyle, et peuvent être substitués une fois par Y-aryle, Y-hétéroaryle, Y-(cycloalkyle en C₃-C₁₂), où l'aryle, l'hétéroaryle ou le cycloalkyle peuvent être substitués une fois à deux fois par, de préférence, un halogène, un alkyle en C₁-C₆, un alkyloxy en C₁-C₆, un hydroxy, un amino, un (alkyle en C₁-C₆)amino, un di (alkyle en C₂-C₁₂)amino, un (alcoxy en C₁-C₆)carbonyle, un (alkyle en C₁-C₆)carbonyle, un cyano, un nitro, un trifluorométhyle, un trifluorométhyloxy ;
X est -CH₂-, qui peut être substitué une fois par un halogène, un méthyle ou un hydroxy ;
Y est une liaison, -O-, -NH- ;
R2, R3, R4, R5 sont, identiques ou différents, un hydrogène, un halogène, un alkyle en C₁-C₆, un trifluorométhyle, un hydroxy, un amino, un cyano, un phénoxy, un (alkyle en C₁-C₆)carbonyle, un (alkyle en C₁-C₆)sulfonyle, un pentafluorosulfanyle, ou un alkyloxy en C₁-C₃ non substitué ou substitué une ou plusieurs fois par F ;
les formes tautomères des composés et leurs sels physiologiquement tolérés.

4. Composé de formule I selon les revendications 1, 2 ou 3, **caractérisé en ce que**
W est -(C=O)- ;
R1 est X-phényle, X-hétéroaryle, ou un bicycle, où l'aryle, l'hétéroaryle ou le bicycle peuvent être substitués une ou plusieurs fois par, de préférence, un halogène, un alkyle en C₁-C₆, un alkyloxy en C₁-C₆, un hydroxy, un (alcoxy en C₁-C₆)carbonyle, un (alkyle en C₁-C₆)carbonyle, un cyano, un nitro, un trifluorométhyle, un trifluorométhyloxy, un (alkyle en C₁-C₆)sulfonyle, un aminosulfonyle, et peuvent être substitués une fois par Y-phényle, Y-hétéroaryle, où le phényle ou l'hétéroaryle peuvent être substitués une fois à deux fois par, de préférence, un halogène, un alkyle en C₁-C₆, un alkyloxy en C₁-C₆, un hydroxy, un amino, un (alcoxy en C₁-C₆)carbonyle, un (alkyle en C₁-C₆)carbonyle, un cyano, un trifluorométhyle, un trifluorométhyloxy ;
X est -CH₂-, qui peut être substitué par un fluor, un méthyle ou un hydroxy ;
Y est une liaison ;
R2, R3, R4, R5 sont, identiques ou différents, un hydrogène, un halogène, un alkyle en C₁-C₆, un trifluorométhyle, un hydroxy, un amino, un cyano, un phénoxy, un (alkyle en C₁-C₆)carbonyle ou un alkyloxy en C₁-C₃ non substitué ou substitué une ou plusieurs fois par F ;
les formes tautomères des composés et leurs sels physiologiquement tolérés.

5. Composé de formule I selon les revendications 1 à 4, **caractérisé en ce que**
W est -(C=O)- ;
R1 est X-phényle, X-hétéroaryle, ou un bicycle de formule Ia 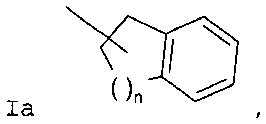 avec n = 1 ou 2, où le phényle, l'hétéroaryle ou le bicycle de formule Ia peuvent être substitués une ou plusieurs fois par, de préférence, un halogène, un alkyle en C₁-C₆, un alkyloxy en C₁-C₆, un hydroxy, un (alcoxy en C₁-C₆)carbonyle, un (alkyle en C₁-C₆)carbonyle, un cyano, un nitro, un trifluorométhyle, un trifluorométhyloxy, un (alkyle en C₁-C₆)sulfonyle, un aminosulfonyle, et peuvent être substitués une fois par Y-phényle, Y-hétéroaryle, où le phényle ou l'hétéroaryle peuvent être substitués une fois à deux fois par, de préférence, un halogène, un alkyle en C₁-C₆, un alkyloxy en C₁-C₆, un hydroxy, un amino, un (alcoxy en C₁-C₆)carbonyle, un (alkyle en C₁-C₆)carbonyle, un cyano, un trifluorométhyle, un trifluorométhyloxy ;
X est -CH₂-, qui peut être substitué par un fluor, un méthyle ou un hydroxy ;
Y est une liaison ;
R2, R3, R4, R5 sont, identiques ou différents, un hydrogène, un halogène, un alkyle en C₁-C₆, un trifluorométhyle, un hydroxy, un amino, un cyano, un phénoxy, un (alkyle en C₁-C₆)carbonyle ou un alkyloxy en C₁-C₃ non substitué ou substitué une ou plusieurs fois par F ;
les formes tautomères des composés et leurs sels physiologiquement tolérés.

6. Composé de formule I selon les revendications 1 à 5, **caractérisé en ce que**
W est -(C=O)- ;
R1 est X-phényle, X-hétéroaryle, ou un bicycle de formule Ia 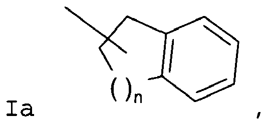 avec n = 1 ou 2, où le phényle, l'hétéroaryle ou le bicycle de formule Ia peuvent être substitués une ou plusieurs fois par, de préférence, un halogène, un alkyle en C₁-C₆, un alkyloxy en C₁-C₆, un hydroxy, un (alcoxy en C₁-C₆)carbonyle, un (alkyle en C₁-C₆)carbonyle, un cyano, un nitro, un trifluorométhyle, un trifluorométhyloxy, un (alkyle en C₁-C₆)sulfonyle, un aminosulfonyle, et peuvent être substitués une fois par Y-phényle, Y-hétéroaryle, où le phényle ou l'hétéroaryle peuvent être substitués une fois à deux fois par, de préférence, un halogène, un alkyle en C₁-C₆, un alkyloxy en C₁-C₆, un hydroxy, un amino, un (alcoxy en C₁-C₆)carbonyle, un (alkyle en C₁-C₆)carbonyle, un cyano, un trifluorométhyle, un trifluorométhyloxy ;
X est -CH₂- qui peut être substitué par un méthyle ;
Y est une liaison ;
R2, R3, R4, R5 sont, identiques ou différents, un hydrogène, un halogène, un alkyle en C₁-C₆, un trifluorométhyle, un hydroxy, un amino, un cyano, un phénoxy, un (alkyle en C₁-C₆)carbonyle ou un alkyloxy en C₁-C₃ non substitué ou substitué une ou plusieurs fois par F.

7. Composé de formule I selon les revendications 1 à 6, **caractérisé en ce que**
W est -(C=O)- ;
R1 est X-phényle, X-thiényle, X-furane, X-benzothiényle, un indanyle ou un tétrahydronaphtyle, où le phényle, le thiényle, le furane, le benzothiényle, l'indanyle ou le tétrahydronaphtyle peuvent être substitués une fois, deux fois ou trois fois par F, Cl, Br, un méthyle, un éthyle, un isopropyle, un méthoxy, un éthoxy, un hydroxy, CO-OCH₃, CO-CH₃, un cyano, un nitro, un trifluorométhyle, un trifluorométhyloxy et
peuvent être substitués une fois par Y-phényle, Y-thiényle, Y-pyridyle, Y-pyrazolyle, où le phényle, le thiényle, le pyridyle ou le pyrazolyle peuvent être substitués une fois à deux fois par, de préférence, F, Cl, Br, un méthyle, un méthoxy, un hydroxy, un amino, CO-OCH₃, CO-CH₃, un cyano, un trifluorométhyle, un trifluorométhyloxy ;
X est -CH₂- qui peut être substitué par un méthyle ;
Y est une liaison ;
R2, R3, R4, R5 sont, identiques ou différents, un hydrogène, F, Cl, un méthoxy, un trifluorométhyle, un cyano, un phénoxy.

8. Composé de formule I selon les revendications 1 à 7, **caractérisé en ce que**
W est -(C=O)- ;
R1 est X-phényle, X-thiényle, X-furane, X-benzothiényle ou un indanyle, où le phényle, le thiényle, X-furane, X-benzothiényle peuvent être substitués une fois ou deux fois par F, Cl, Br, un méthyle, un isopropyle, un méthoxy, un cyano, un trifluorométhyle et peuvent être substitués une fois par Y-phényle, Y-thiényle, Y-pyridyle, Y-pyrazolyle, où le phényle peut être substitué par Cl ;
X est -CH₂-, qui peut être substitué par un méthyle ;
Y est une liaison ;
R2 est un hydrogène, F, Cl ;
R3 est un hydrogène, F, Cl, un méthoxy, un trifluorométhyle, un cyano, un phénoxy ;
R4 est un hydrogène, F, Cl, un méthoxy, un trifluorométhyle, un cyano, un phénoxy ;
R5 est un hydrogène, F, Cl.

9. Composé de formule I selon les revendications 1 à 8, **caractérisé en ce que**
R2, R3, R4, R5 sont un hydrogène.

10. Composé de formule I selon les revendications 1 à 8, **caractérisé en ce que**
R2, R3, R5 sont un hydrogène ; et
R4 n'est pas un hydrogène ; ou
R2, R4, R5 sont un hydrogène ;
et
R3 n'est pas un hydrogène.

11. Médicament comprenant un ou plusieurs composés de formule I selon les revendications 1 à 10.

12. Utilisation des composés de formule I selon les revendications 1 à 10 pour la fabrication d'un médicament pour le traitement et/ou la prévention de troubles du métabolisme des acides gras et de troubles d'utilisation du glucose.

13. Utilisation des composés de formule I selon les revendications 1 à 10 pour la fabrication d'un médicament pour le traitement et/ou la prévention de troubles dans lesquels l'insulinorésistance est impliquée.

14. Utilisation des composés de formule I selon les revendications 1 à 10 pour la fabrication d'un médicament pour le traitement et/ou la prévention du diabète sucré et des séquelles associées à celui-ci.

15. Utilisation des composés de formule I selon les revendications 1 à 10 pour la fabrication d'un médicament pour le traitement et/ou la prévention de dyslipidémies et leurs séquelles.

16. Utilisation des composés de formule I selon les revendications 1 à 10 pour la fabrication d'un médicament pour le traitement et/ou la prévention de pathologies associées au syndrome métabolique.

17. Utilisation des composés de formule I selon les revendications 1 à 10 pour la fabrication d'un médicament pour le traitement et/ou la prévention de pathologies associées à un taux de HDL réduit.

18. Utilisation des composés de formule I selon les revendications 1 à 10 pour la fabrication d'un médicament pour le traitement et/ou la prévention de troubles athérosclérotiques.

19. Utilisation des composés de formule I selon les revendications 1 à 10 en combinaison avec au moins un ingrédient actif supplémentaire pour la fabrication d'un médicament pour le traitement et/ou la prévention de troubles dans lesquels l'insulinorésistance est impliquée.

20. Procédé pour fabriquer un médicament comprenant un ou plusieurs des composés de formule I selon les revendications 1 à 10, **caractérisé en ce qu'**il comprend le mélange de ceux-ci avec un véhicule pharmaceutiquement adapté et la conversion de ce mélange en une forme adaptée pour administration.

21. Procédé de préparation de composés de formule générale I selon les revendications 1 à 10, **caractérisé en ce que** des dérivés de benzotriazole de formule II
a) sont acylés avec des chlorures de carbamoyle de formule III ;
ou
b) en deux étapes réagissent dans un premier temps avec du phosgène ou des équivalents tels que le chlorocarbonate de trichlorométhyle, le carbonate de ditrichlorométhyle ou le chloroformate de 4-nitrophényle et dans une seconde étape avec des amines de formule IV,
ou
c) réagissent avec des isocyanates de formule V : O=C=N-R1,
dans lesquels les substituants ont les définitions mentionnées ci-dessus, et
le cas échéant les régio-isomères résultants sont séparés par des procédés connus, en particulier des procédés chromatographiques.
